# EUROPEAN PATENT APPLICATION

(11) **EP 2 762 161 A1**
(43) Date of publication of application: **06.08.2014**
(21) Application number: 14000326.0
(22) Date of filing: 29.01.2014
(51) Int. Cl.: A61K 39/00

(54) **E75-vaccine composition for mucosal administration**

(30) Priority: 05.02.2013 JP 2013020909
(71) Applicant: NITTO DENKO CORPORATION, Ibaraki-shi, Osaka 567-8680 (JP)
(72) Inventor: Asari, Daisuke, Ibaraki-shi, Osaka 567-8680 (JP); Okazaki, Arimichi, Ibaraki-shi, Osaka 567-8680 (JP); Matsushita, Kyohei, Ibaraki-shi, Osaka 567-8680 (JP); Okubo, Katsuyuki, Ibaraki-shi, Osaka 567-8680 (JP); Maeda, Yoshiki, Ibaraki-shi, Osaka 567-8680 (JP); Shishido, Takuya, Ibaraki-shi, Osaka 567-8680 (JP); Li, Wenjing, Ibaraki-shi, Osaka 567-8680 (JP); Hori, Mitsuhiko, Ibaraki-shi, Osaka 567-8680 (JP); Sugiyama, Haruo, Suita-shi, Osaka 565-0871 (JP)
(74) Representative: Müller-Boré & Partner Patentanwälte PartG mbB

(57) **Abstract**

The present invention provides a cancer vaccine composition for mucosal administration comprising (i) a HER2/neu E75 peptide and/or a modified HER2/neu E75 peptide; and (ii) a first cellular immunity induction promoter.

SEQUENCE LISTING

<110>  NITTO DENKO CORPORATION

<120>  VACCINE COMPOSITION FOR MUCOSAL ADMINISTRATION

<130>  NITTO 8

<150>  JP 2013-020909
<151>  2013-02-05

<160>  4

<170>  PatentIn version 3.2

<210>  1
<211>  9
<212>  PRT
<213>  Homo sapiens

<400>  1

Lys Ile Phe Gly Ser Leu Ala Phe Leu
1               5


<210>  2
<211>  20
<212>  DNA
<213>  Unknown

<220>
<223>  bacterial DNA

<400>  2
tccatgacgt tcctgacgtt                                                    20


<210>  3
<211>  15
<212>  PRT
<213>  Mycobacterium tuberculosis

<400>  3

Phe Gln Asp Ala Tyr Asn Ala Ala Gly Gly His Asn Ala Val Phe
1               5                   10                  15


<210>  4
<211>  13
<212>  PRT

<213>  Artificial sequence

<220>
<223>  Synthetic peptide

<220>
<221>  MOD_RES
<222>  (1)..(1)
<223>  D-alanine

<220>
<221>  MOD_RES
<222>  (3)..(3)
<223>  cyclohexylalanine

<220>
<221>  MOD_RES
<222>  (13)..(13)
<223>  D-alanine

<400>  4

Ala Lys Ala Val Ala Ala Trp Thr Leu Lys Ala Ala Ala
1               5                   10

## Description

### TECHNICAL FIELD

The present invention relates to a cancer vaccine for mucosal administration comprising a HER2 antigen peptide, particularly a HER2/neu E75 peptide and/or a modified HER2/neu E75 peptide, and a cellular immunity induction promoter.

### BACKGROUND ART

There are a cancer vaccine that prevents viral infection to prevent a cancer caused by the virus, and a cancer vaccine which provides the result that cancer cells are specifically attacked by the immune system via the recognition of a cancer-specific antigen by the immune mechanism, particularly the cellular immune mechanism in which cytotoxic T cells (CTLs) play an important role. The former is not effective at all for a cancer in which the virus does not participate. The latter is a cancer therapeutic strategy of targeting an antigen possessed by a cancer cell itself. It is considered that the latter is widely effective for cancers having antigen by specifying the antigen. Inter alia, a cancer vaccine based on the viewpoint of the latter can treat tumors that are difficult to remove by surgical operation because of their size, and causes less side effects as compared with the conventional therapies such as chemotherapy and radiation therapy.

A HER2/neu gene is amplified and overexpressed in various adenocarcinomas including breast cancer. Those cancers overproduce the HER2/neu protein. The HER2/neu protein is fragmented in the cancer cell to produce partial peptides containing 8 to 12 amino acids. A HER2/neu peptide is one of the peptide fragment which has been bound with the MHC class I molecule in a cancer cell, moved to the surface of the cancer cell, and presented as an antigen bound to the MHC class I molecule on the cancer cell surface. The peptide becomes a mark of the cancer cell. The HER2/neu E75 peptide, which is a HLA-A2 or A3 haplotype MHC restricted peptide, is considered as a particularly promising HER2/neu peptide because it is reported to be effective in clinical tests. The amino acid sequence of the HER2/neu E75 peptide is Lys-Ile-Phe-Gly-Ser-Leu-Ala-Phe-Leu (SEQ ID NO: 1). When HER2/neu E75 peptide, or a modified HER2/neu E75 peptide in which a part of amino acids of the HER2/neu E75 peptide is substituted or modified is administered to a living body as an antigen (herein, the HER2/neu E75 peptide or modified HER2/neu E75 peptide which has been administered as an antigen is referred to as "E75 antigen peptide"), the E75 antigen peptide is bound to the MHC class I molecule on the surface of a dendritic cell which is an antigen presenting cell, or the E75 antigen peptide is once taken into a dendritic cell, bound to the MHC class I molecule of the dendritic cell, and then, is moved to the surface of the dendritic cell, thereby, is presented as an antigen bound to the MHC class I molecule on the surface of the dendritic cell. An activated dendritic cell having the E75 antigen peptide/MHC class I molecule complex is moved to the regional lymph node, and activates a CD8-positive T lymphocyte which recognizes the E75 antigen peptide/MHC class I molecule complex to differentiate and proliferate the CD8-positive T lymphocyte into a cytotoxic T-cell (CTL). The CTL recognizes tumor cells having the complex of HER2/neu E75 peptide (derived from the endogenous HER2/neu protein) of the same amino acid sequence as the E75 antigen peptide and the MHC class I molecule; or a tumor cell having a complex of HER2/neu E75 peptide (derived from the endogenous HER2/neu protein) of an amino acid sequence having cross immunoreactivity with the E75 antigen peptide and the MHC class I molecule, and attacks the recognized tumor cells. Therefore, the HER2/neu E75 peptide, and the modified HER2/neu E75 peptide in which a part of amino acids thereof are substituted or modified are useful as cancer vaccines.

It is also known that an adjuvant is utilized in order to enhance the action as cancer vaccine of the HER2/neu E75 peptide. The HER2/neu E75 peptide has been investigated and reported to involve immunization by injection, and GM-CSF is known as an adjuvant to be used on that occasion.

In general, vaccines are administered by subcutaneous or intradermal injection. In addition to those routes, immunity induction by a various of administration route, for example, transdermal administration (Patent Document 1 and Non-Patent Document 1); and mucosal administration such as buccal administration, nasal administration, and sublingual administration (Non-Patent Document 2, Patent Document 2, and Patent Document 3) have been tried.

### LIST OF DOCUMENTS

[Patent Document 1] US Patent Application Publication No. US 2008/0193487
[Patent Document 2] JP 2002-531415 A
[Patent Document 3] US Patent Application Publication No. US 2008/0112974
[Patent Document 4] JP 7-505883 A
[Patent Document 5] JP 2007-529531 A

[Non-Patent Document 1] Hosoi Akihiro et al., Cancer Research, 68, 3941-3949 (2008)
[Non-Patent Document 2] Zhengrong Cui et al., Pharmaceutical Research, Vol. 19, No. 7, 947-953 (2002)
[Non-Patent Document 3] Mittendorf EA et al., Cancer Immunol Immunother., 57(10), 1511-1521 (2008)
[Non-Patent Document 4] George EP et al., Journal of Clinical Oncology, 23(30), 7536-7545 (2005)

### SUMMARY OF THE INVENTION

It is well known that an adjuvant is used to enhance efficacy of a vaccine. Suitable adjuvant generally vary depending on, for example, the kind of the antigen, the administration route, and the immune response which is desired to be induced (that is, cellular immunity or humoral immunity). Further, in addition to the adjuvant, there are a variety of substances which promote the induction of the immunity. Thus, an object of the present invention is to provide a composition for use as a cancer vaccine, which has higher efficacy and is convenient for use.

A microorganism or a virus itself, or a part of them is contained in a widely used vaccine and the vaccine is administered to induce immune response. Usually, since invasion of the microorganism or virus is inhibited by the skin due to the size thereof, it is difficult that the vaccine is administered on the skin. Furthermore, it is also difficult to administer the vaccine orally because the microorganism or virus is decomposed by gastric acid and digestive enzymes. Therefore, injectable formulations which are invasively administered into the body have been generally utilized. However, the injection has some problems including pain, fear, injection scar, and subsequent scarring cicatrization. People other than health care workers are not permitted to perform the injection. Intradermal injection which can introduce higher immune response is a difficult administration technique. There is a risk of accidental infection of the health care workers due to needlestick injury. Patients are needed to visit the hospital repeatedly when administration is performed repetitively. Medical wastes which necessitate special disposition such as injection needles are generated. In view of the above issues, injection is not necessarily the optimal administration route.

A HER2/neu E75 peptide and/or a modified HER2/neu E75 peptide can activate CTLs (cytotoxic T-cells) via MHC class I molecules. Furthermore, since HER2/neu E75 peptide is a molecule consisting of 9 amino acids and having a molecular weight of about 1000, and is significantly smaller than microorganisms or virus itself although they are not considered as a small molecular substance. It may be possible that they are administered by a route other than injection. However, a preparation for the administration of the peptide vaccine in a rout other than injection has not been developed yet. The reason includes many things, for example: a suitable substance that can promote to induce the cellular immunity has been unknown; it has also been unknown whether or not an antigen can be delivered to a tissue suitable for the induction of the cellular immunity. Inter alia, a substance that can promote to induce the cellular immunity when it is used with the antigen when administered in a route other than injection has been unknown.

The inventors have found that the immunity can effectively be induced by mucosal administration of the peptide vaccine. The inventors also found some substances suitable for enhancing immunity induced by the mucosal administration of a HER2/neu E75 peptide and/or a modified HER2/neu E75 peptide. The substances may include TLR ligands such as Pam₃CSK₄, poly(I:C), lipopolysaccharides, imiquimod and resiquimod; cyclic dinucleotides such as cyclic di-GMP and cyclic di-AMP; immunomodulatory small molecule drugs such as levamisole hydrochloride; cyclooxygenase inhibitors such as etodolac and loxoprofen; prostaglandin receptor antagonists such as EP2 receptor antagonists, EP4 receptor antagonists, DP receptor antagonists, and IP receptor antagonists; prostaglandin receptor agonists such as EP3 receptor agonists; TSLP production inhibitors such as berberine chloride and naringenin; adenylate cyclase inhibitors such as 2',5'-dideoxyadenosine and niacin; omega-3 fatty acids such as eicosapentaenoic acid and docosahexaenoic acid; PPAR agonists such as PPAR-α agonists, PPAR-δ agonists, and PPAR-γ agonists; dopamine receptor antagonists such as D1 receptor antagonists and D5 receptor antagonists; dopamine receptor agonists such as D2 receptor agonists, D3 receptor agonists, and D4 receptor agonists; histamine receptor antagonists such as H1 receptor antagonists and H2 receptor antagonists; histamine receptor agonists such as H1 receptor agonists, H3 receptor agonists, and H4 receptor agonists; serotonin receptor antagonists such as 5-HT2 receptor antagonists, 5-HT4 receptor antagonists, 5-HT6 receptor antagonists, and 5-HT7 receptor antagonists; serotonin receptor agonists such as 5-HT1 receptor agonists and 5-HT2 receptor agonists; vasopressin receptor antagonists such as V2 receptor antagonists; vasopressin receptor agonists such as V1 receptor agonists; muscarine receptor antagonists such as M1 receptor antagonists, M3 receptor antagonists, and M5 receptor antagonists; muscarine receptor agonists such as M1 receptor agonists, M2 receptor agonists, M3 receptor agonists, M4 receptor agonists, and M5 receptor agonists; adrenaline receptor antagonists such as α1 receptor antagonists, β1 receptor antagonists, β2 receptor antagonists, and β3 receptor antagonists; adrenaline receptor agonists such as α1 receptor agonists and α2 receptor agonists; angiotensin receptor agonists such as AT2 receptor agonists; GABA receptor agonists such as GABA_{B} receptor agonists; thrombin receptor antagonists such as PAR-1 receptor antagonists; thrombin receptor agonists such as PAR-1 receptor agonists; opioid receptor agonists such as buprenorphine; leukotriene receptor antagonists such as CysLT1 receptor antagonists and CysLT2 receptor antagonists; leukotriene receptor agonists such as BLT receptor agonists; ADP receptor agonists such as adenosine diphosphate; melatonin receptor agonists such as melatonin; somatostatin receptor agonists such as octreotide; cannabinoid receptor agonists such as dronabinol; sphingosine-1 phosphate receptor agonists such as fingolimod; metabotropic glutamate receptor agonists such as mGluR2 receptor agonists, mGluR3 receptor agonists, mGluR4 receptor agonists, mGluR6 receptor agonists, mGluR7 receptor agonists, and mGluR8 receptor agonists; phospholipase A2 inhibitors such as glycyrrhizic acid; TGF-β production inhibitors such as pirfenidone; Th2 cytokine inhibitors such as suplatast tosylate; pharmacologically acceptable acids such as decanoic acid, lauric acid, myristic acid, isostearic acid and oleic acid, or pharmacologically acceptable salts thereof; and helper peptides such as Peptide-25.

In regard to oral mucosal administration, it was also found that it is particularly suitable to use TLR1/2 ligands such as Pam₃CSK₄; TLR7 and/or TLR8 ligands such as imiquimod and resiquimod; cyclic dinucleotides such as cyclic di-GMP and cyclic di-AMP; immunomodulatory small molecule drugs such as levamisole hydrochloride; cyclooxygenase inhibitors such as etodolac and loxoprofen; prostaglandin receptor antagonists such as EP2 receptor antagonists, EP4 receptor antagonists, DP receptor antagonists, and IP receptor antagonists; prostaglandin receptor agonists such as EP3 receptor agonists; TSLP production inhibitors such as berberine chloride and naringenin; adenylate cyclase inhibitors such as 2',5'-dideoxyadenosine and niacin; omega-3 fatty acids such as eicosapentaenoic acid and docosahexaenoic acid; PPAR agonists such as PPAR-α agonists, PPAR-δ agonists and PPAR-γ agonists; dopamine receptor antagonists such as D1 receptor antagonists and D5 receptor antagonists; dopamine receptor agonists such as D2 receptor agonists, D3 receptor agonists, and D4 receptor agonists; histamine receptor antagonists such as H1 receptor antagonists and H2 receptor antagonists; histamine receptor agonists such as H1 receptor agonists, H3 receptor agonists, and H4 receptor agonists; serotonin receptor antagonists such as 5-HT2 receptor antagonists, 5-HT4 receptor antagonists, 5-HT6 receptor antagonists and 5-HT7 receptor antagonists; serotonin receptor agonists such as 5-HT1 receptor agonists and 5-HT2 receptor agonists; vasopressin receptor antagonists such as V2 receptor antagonists; vasopressin receptor agonists such as V1 receptor agonists; muscarine receptor antagonists such as M1 receptor antagonists, M3 receptor antagonists, and M5 receptor antagonists; muscarine receptor agonists such as M1 receptor agonists, M2 receptor agonists, M3 receptor agonists, M4 receptor agonists, and M5 receptor agonists; adrenaline receptor antagonists such as α1 receptor antagonists, β1 receptor antagonists, β2 receptor antagonists, and β3 receptor antagonists; adrenaline receptor agonists such as α1 receptor agonists and α2 receptor agonists; angiotensin receptor agonists such as AT2 receptor agonists; GABA receptor agonists such as GABA_{B} receptor agonists; thrombin receptor antagonists such as PAR-1 receptor antagonists; thrombin receptor agonists such as PAR-1 receptor agonists; opioid receptor agonists such as buprenorphine; leukotriene receptor antagonists such as CysLT1 receptor antagonists and CysLT2 receptor antagonists; leukotriene receptor agonists such as BLT receptor agonists; ADP receptor agonists such as adenosine diphosphate; melatonin receptor agonists such as melatonin; somatostatin receptor agonists such as octreotide; cannabinoid receptor agonists such as dronabinol; sphingosine-1 phosphate receptor agonists such as fingolimod; metabotropic glutamate receptor agonists such as mGluR2 receptor agonists, mGluR3 receptor agonists, mGluR4 receptor agonists, mGluR6 receptor agonists, mGluR7 receptor agonists, and mGluR8 receptor agonists; phospholipase A2 inhibitors such as glycyrrhizic acid; TGF-β production inhibitors such as pirfenidone; Th2 cytokine inhibitors such as suplatast tosylate; helper peptides; or combinations of two or more of them. Furthermore, it was also found that cellular immunity is markedly enhanced by a combination of a TLR ligand and a helper peptide, a combination of a cyclic dinucleotide and a helper peptide, a combination of an immunomodulatory small molecule drug and a helper peptide, a combination of a cyclooxygenase inhibitor and a helper peptide, a combination of a prostaglandin receptor antagonist and a helper peptide, a combination of a prostaglandin receptor agonist and a helper peptide, a combination of a TSLP production inhibitor and a helper peptide, a combination of an adenylate cyclase inhibitor and a helper peptide, a combination of an omega-3 fatty acid and a helper peptide, a combination of a PPAR agonist and a helper peptide, a combination of a dopamine receptor antagonist and a helper peptide, a combination of a dopamine receptor agonist and a helper peptide, a combination of a histamine receptor agonist and a helper peptide, a combination of a histamine receptor antagonist and a helper peptide, a combination of a serotonin receptor agonist and a helper peptide, a combination of a serotonin receptor antagonist and a helper peptide, a combination of a vasopressin receptor antagonist and a helper peptide, a combination of a vasopressin receptor agonist and a helper peptide, a combination of a muscarine receptor antagonist and a helper peptide, a combination of a muscarine receptor agonist and a helper peptide, a combination of an adrenaline receptor antagonist and a helper peptide, a combination of an adrenaline receptor agonist and a helper peptide, a combination of an angiotensin receptor agonist and a helper peptide, a combination of a GABA receptor agonist and a helper peptide, a combination of a thrombin receptor antagonist and a helper peptide, a combination of a thrombin receptor agonist and a helper peptide, a combination of an opioid receptor agonist and a helper peptide, a combination of an ADP receptor agonist and a helper peptide, a combination of a leukotriene receptor antagonist and a helper peptide, a combination of a leukotriene receptor agonist and a helper peptide, a combination of a melatonin receptor agonist and a helper peptide, a combination of a somatostatin receptor agonist and a helper peptide, a combination of a cannabinoid receptor agonist and a helper peptide, a combination of a sphingosine-1 phosphate receptor agonist and a helper peptide, a combination of a metabotropic glutamate receptor agonist and a helper peptide, a combination of a phospholipase A2 inhibitor and a helper peptide, a combination of a TGF-β production inhibitor and a helper peptide, a combination of a Th2 cytokine inhibitor and a helper peptide, and a combination of a pharmacologically acceptable acid or a pharmacologically acceptable salt thereof and a helper peptide.

In regard to nasal mucosal administration, it was found that it is particularly suitable to use TLR1/2 ligands such as Pam₃CSK₄; TLR3 ligands such as poly(I:C); TLR4 ligands such as lipopolysaccharides; cyclic dinucleotides such as cyclic di-GMP and cyclic di-AMP; cyclooxygenase inhibitors such as etodolac and loxoprofen; prostaglandin receptor antagonists such as EP2 receptor antagonists, EP4 receptor antagonists, DP receptor antagonists, and IP receptor antagonists; prostaglandin receptor agonists such as EP3 receptor agonists; TSLP production inhibitors such as berberine chloride and naringenin; adenylate cyclase inhibitors such as 2',5'-dideoxyadenosine and niacin; omega-3 fatty acids such as eicosapentaenoic acid and docosahexaenoic acid; PPAR agonists such as PPAR-α agonists, PPAR-δ agonists and PPAR-γ agonists; dopamine receptor antagonists such as D1 receptor antagonists and D5 receptor antagonists; dopamine receptor agonists such as D2 receptor agonists, D3 receptor agonists, and D4 receptor agonists; histamine receptor antagonists such as H1 receptor antagonists and H2 receptor antagonists; histamine receptor agonists such as H1 receptor agonists, H3 receptor agonists, and H4 receptor agonists; serotonin receptor antagonists such as 5-HT2 receptor antagonists, 5-HT4 receptor antagonists, 5-HT6 receptor antagonists and 5-HT7 receptor antagonists; serotonin receptor agonists such as 5-HT1 receptor agonists and 5-HT2 receptor agonists; vasopressin receptor antagonists such as V2 receptor antagonists; vasopressin receptor agonists such as V1 receptor agonists; muscarine receptor antagonists such as M1 receptor antagonists, M3 receptor antagonists, and M5 receptor antagonists; muscarine receptor agonists such as M1 receptor agonists, M2 receptor agonists, M3 receptor agonists, M4 receptor agonists, and M5 receptor agonists; adrenaline receptor antagonists such as α1 receptor antagonists, β1 receptor antagonists, β2 receptor antagonists, and β3 receptor antagonists; adrenaline receptor agonists such as α1 receptor agonists and α2 receptor agonists; angiotensin receptor agonists such as AT2 receptor agonists; GABA receptor agonists such as GABA_{B} receptor agonists; thrombin receptor antagonists such as PAR-1 receptor antagonists; thrombin receptor agonists such as PAR-1 receptor agonists; opioid receptor agonists such as buprenorphine; leukotriene receptor antagonists such as CysLT1 receptor antagonists and CysLT2 receptor antagonists; leukotriene receptor agonists such as BLT receptor agonists; ADP receptor agonists such as adenosine diphosphate; melatonin receptor agonists such as melatonin; somatostatin receptor agonists such as octreotide; cannabinoid receptor agonists such as dronabinol; sphingosine-1 phosphate receptor agonists such as fingolimod; metabotropic glutamate receptor agonists such as mGluR2 receptor agonists, mGluR3 receptor agonists, mGluR4 receptor agonists, mGluR6 receptor agonists, mGluR7 receptor agonists, and mGluR8 receptor agonists; phospholipase A2 inhibitors such as glycyrrhizic acid; TGF-β production inhibitors such as pirfenidone; Th2 cytokine inhibitors such as suplatast tosylate; helper peptides; or combinations of two or more of them.

Furthermore, it was also found that cellular immunity is markedly enhanced by a combination of a TLR ligand and a helper peptide, a combination of a cyclic dinucleotide and a helper peptide, a combination of an immunomodulatory small molecule drug and a helper peptide, a combination of a cyclooxygenase inhibitor and a helper peptide, a combination of a prostaglandin receptor antagonist and a helper peptide, a combination of a prostaglandin receptor agonist and a helper peptide, a combination of a TSLP production inhibitor and a helper peptide, a combination of an adenylate cyclase inhibitor and a helper peptide, a combination of an omega-3 fatty acid and a helper peptide, a combination of a PPAR agonist and a helper peptide, a combination of a dopamine receptor antagonist and a helper peptide, a combination of a dopamine receptor agonist and a helper peptide, a combination of a histamine receptor agonist and a helper peptide, a combination of a histamine receptor antagonist and a helper peptide, a combination of a serotonin receptor agonist and a helper peptide, a combination of a serotonin receptor antagonist and a helper peptide, a combination of a vasopressin receptor antagonist and a helper peptide, a combination of a vasopressin receptor agonist and a helper peptide, a combination of a muscarine receptor antagonist and a helper peptide, a combination of a muscarine receptor agonist and a helper peptide, a combination of an adrenaline receptor antagonist and a helper peptide, a combination of an adrenaline receptor agonist and a helper peptide, a combination of an angiotensin receptor agonist and a helper peptide, a combination of a GABA receptor agonist and a helper peptide, a combination of a thrombin receptor antagonist and a helper peptide, a combination of a thrombin receptor agonist and a helper peptide, a combination of an opioid receptor agonist and a helper peptide, a combination of an ADP receptor agonist and a helper peptide, a combination of a leukotriene receptor antagonist and a helper peptide, a combination of a leukotriene receptor agonist and a helper peptide, a combination of a melatonin receptor agonist and a helper peptide, a combination of a somatostatin receptor agonist and a helper peptide, a combination of a cannabinoid receptor agonist and a helper peptide, a combination of a sphingosine-1 phosphate receptor agonist and a helper peptide, a combination of a metabotropic glutamate receptor agonist and a helper peptide, a combination of a phospholipase A2 inhibitor and a helper peptide, a combination of a TGF-β production inhibitor and a helper peptide, a combination of Th2 cytokine inhibitor and a helper peptide, and a combination of a pharmacologically acceptable acid or a pharmacologically acceptable salt thereof and a helper peptide.

Therefore, the present invention, in a first aspect, provides the aspects listed below:
(1) a cancer vaccine composition for mucosal administration, comprising:
   (i) a HER2/neu E75 peptide and/or a modified HER2/neu E75 peptide; and
   (ii) a first cellular immunity induction promoter selected from a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenaline receptor antagonist, an adrenaline receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor, a Th2 cytokine inhibitor, and combinations of two or more of them;
(2) the cancer vaccine composition for mucosal administration according to (1), further comprising a pharmacologically acceptable acid or a pharmacologically acceptable salt thereof as a second cellular immunity induction promoter;
(3) the cancer vaccine composition for mucosal administration according to (1) or (2), wherein the first cellular immunity induction promoter is a TLR ligand;
(4) the cancer vaccine composition for mucosal administration according to (1) or (2), wherein the first cellular immunity induction promoter is a cyclic dinucleotide;
(5) the cancer vaccine composition for mucosal administration according to (1) or (2), wherein the first cellular immunity induction promoter is an immunomodulatory small molecule drug;
(6) the cancer vaccine composition for mucosal administration according to (1) or (2), wherein the first cellular immunity induction promoter is a cyclooxygenase inhibitor;
(7) the cancer vaccine composition for mucosal administration according to (1) or (2), wherein the first cellular immunity induction promoter is a prostaglandin receptor antagonist, and the prostaglandin receptor antagonist is an EP2 receptor antagonist, an EP4 receptor antagonist, a DP receptor antagonist or an IP receptor antagonist;
(8) the cancer vaccine composition for mucosal administration according to (1) or (2), wherein the first cellular immunity induction promoter is a prostaglandin receptor agonist, and the prostaglandin receptor agonist is an EP3 receptor agonist;
(9) the cancer vaccine composition for mucosal administration according to (1) or (2), wherein the first cellular immunity induction promoter is a TSLP production inhibitor;
(10) the cancer vaccine composition for mucosal administration according to (1) or (2), wherein the first cellular immunity induction promoter is an adenylate cyclase inhibitor;
(11) the cancer vaccine composition for mucosal administration according to (1) or (2), wherein the first cellular immunity induction promoter is an omega-3 fatty acid;
(12) the cancer vaccine composition for mucosal administration according to (1) or (2), wherein the first cellular immunity induction promoter is a PPAR agonist, and the PPAR agonist is a PPAR-α agonist, a PPAR-δ agonist, or a PPAR-γ agonist;
(13) the cancer vaccine composition for mucosal administration according to (1) or (2), wherein the first cellular immunity induction promoter is a dopamine receptor antagonist, and the dopamine receptor antagonist is a D1 receptor antagonist or a D5 receptor antagonist;
(14) the cancer vaccine composition for mucosal administration according to (1) or (2), wherein the first cellular immunity induction promoter is a dopamine receptor agonist, and the dopamine receptor agonist is a D2 receptor agonist, a D3 receptor agonist, or a D4 receptor agonist;
(15) the cancer vaccine composition for mucosal administration according to (1) or (2), wherein the first cellular immunity induction promoter is a histamine receptor antagonist, and the histamine receptor antagonist is a H1 receptor antagonist or a H2 receptor antagonist;
(16) the cancer vaccine composition for mucosal administration according to (1) or (2), wherein the first cellular immunity induction promoter is a histamine receptor agonist, and the histamine receptor agonist is a H1 receptor agonist, a H3 receptor agonist or a H4 receptor agonist;
(17) the cancer vaccine composition for mucosal administration according to (1) or (2), wherein the first cellular immunity induction promoter is a serotonin receptor antagonist, and the serotonin receptor antagonist is a 5-HT2 receptor antagonist, a 5-HT4 receptor antagonist, a 5-HT6 receptor antagonist, or a 5-HT7 receptor antagonist;
(18) the cancer vaccine composition for mucosal administration according to (1) or (2), wherein the first cellular immunity induction promoter is a serotonin receptor agonist, and the serotonin receptor agonist is a 5-HT1 receptor agonist or a 5-HT2 receptor agonist;
(19) the cancer vaccine composition for mucosal administration according to (1) or (2), wherein the first cellular immunity induction promoter is a vasopressin receptor antagonist, and the vasopressin receptor antagonist is a V2 receptor antagonist;
(20) the cancer vaccine composition for mucosal administration according to (1) or (2), wherein the first cellular immunity induction promoter is a vasopressin receptor agonist, and the vasopressin receptor agonist is a V1 receptor agonist;
(21) the cancer vaccine composition for mucosal administration according to (1) or (2), wherein the first cellular immunity induction promoter is a muscarine receptor antagonist, and the muscarine receptor antagonist is a M1 receptor antagonist, a M3 receptor antagonist, or a M5 receptor antagonist;
(22) the cancer vaccine composition for mucosal administration according to (1) or (2), wherein the first cellular immunity induction promoter is a muscarine receptor agonist, and the muscarine receptor agonist is a M1 receptor agonist, a M2 receptor agonist, a M3 receptor agonist, a M4 receptor agonist, or a M5 receptor agonist;
(23) the cancer vaccine composition for mucosal administration according to (1) or (2), wherein the first cellular immunity induction promoter is an adrenaline receptor antagonist, and the adrenaline receptor antagonist is an α1 receptor antagonist, a β1 receptor antagonist, a β2 receptor antagonist, or a β3 receptor antagonist;
(24) the cancer vaccine composition for mucosal administration according to (1) or (2), wherein the first cellular immunity induction promoter is an adrenaline receptor agonist, and the adrenaline receptor agonist is an α1 receptor agonist or an α2 receptor agonist;
(25) the cancer vaccine composition for mucosal administration according to (1) or (2), wherein the first cellular immunity induction promoter is an angiotensin receptor agonist, and the angiotensin receptor agonist is an AT2 receptor agonist;
(26) the vaccine composition for mucosal administration according to (1) or (2), wherein the first cellular immunity induction promoter is a GABA receptor agonist, and the GABA receptor agonist is a GABA_{B} receptor agonist;
(27) the cancer vaccine composition for mucosal administration according to (1) or (2), wherein the first cellular immunity induction promoter is a thrombin receptor antagonist, and the thrombin receptor antagonist is a PAR-1 receptor antagonist;
(28) the cancer vaccine composition for mucosal administration according to (1) or (2), wherein the first cellular immunity induction promoter is a thrombin receptor agonist, and the thrombin receptor agonist is a PAR-1 receptor agonist;
(29) the cancer vaccine composition for mucosal administration according to (1) or (2), wherein the first cellular immunity induction promoter is an opioid receptor agonist;
(30) the cancer vaccine composition for mucosal administration according to (1) or (2), wherein the first cellular immunity induction promoter is a leukotriene receptor antagonist, and the leukotriene receptor antagonist is a CysLT1 receptor antagonist or a CysLT2 receptor antagonist;
(31) the cancer vaccine composition for mucosal administration according to (1) or (2), wherein the first cellular immunity induction promoter is a leukotriene receptor agonist, and the leukotriene receptor agonist is a BLT receptor agonist;
(32) the cancer vaccine composition for mucosal administration according to (1) or (2), wherein the first cellular immunity induction promoter is a melatonin receptor agonist;
(33) the cancer vaccine composition for mucosal administration according to (1) or (2), wherein the first cellular immunity induction promoter is a somatostatin receptor agonist;
(34) the cancer vaccine composition for mucosal administration according to (1) or (2), wherein the first cellular immunity induction promoter is a cannabinoid receptor agonist;
(35) the cancer vaccine composition for mucosal administration according to (1) or (2), wherein the first cellular immunity induction promoter is a sphingosine-1 phosphate receptor agonist;
(36) the cancer vaccine composition for mucosal administration according to (1) or (2), wherein the first cellular immunity induction promoter is a metabotropic glutamate receptor agonist, and the metabotropic glutamate receptor agonist is a mGluR2 receptor agonist, a mGluR3 receptor agonist, a mGluR4 receptor agonist, a mGluR6 receptor agonist, a mGluR7 receptor agonist, or a mGluR8 receptor agonist;
(37) the cancer vaccine composition for mucosal administration according to (1) or (2), wherein the first cellular immunity induction promoter is an ADP receptor agonist;
(38) the cancer vaccine composition for mucosal administration according to (1) or (2), wherein the first cellular immunity induction promoter is a phospholipase A2 inhibitor;
(39) the cancer vaccine composition for mucosal administration according to (1) or (2), wherein the first cellular immunity induction promoter is a TGF-β production inhibitor;
(40) the cancer vaccine composition for mucosal administration according to (1) or (2), wherein the first cellular immunity induction promoter is a Th2 cytokine inhibitor;
(41) the cancer vaccine composition for mucosal administration according to (1) or (2), wherein the first cellular immunity induction promoter is a helper peptide;
(42) the cancer vaccine composition for mucosal administration according to (1) or (2), wherein the first cellular immunity induction promoter is a combination of a helper peptide and one or more substances selected from the group consisting of a TLR ligand, a cyclic dinucleotide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenaline receptor antagonist, an adrenaline receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor and a Th2 cytokine inhibitor;
(43) a cancer vaccine film formulation for mucosal administration, comprising the pharmaceutical composition according to any one of (1) to (42) and being in the form of a film formulation;
(44) a cancer vaccine liquid formulation for mucosal administration, comprising the pharmaceutical composition according to any one of (1) to (42) and being in the form of a liquid formulation; and
(45) a cancer vaccine orally-disintegrating tablet for mucosal administration, comprising the pharmaceutical composition according to any one of (1) to (42) and being in the form of an orally-disintegrating tablet.

According to another aspect, the cancer vaccine composition of the present invention can be used for the treatment or prevention of cancer. Therefore, the present invention also provides the following embodiments:
(46) a method for treating or preventing cancer in a subject, comprising mucosally administering to the subject a therapeutically effective amounts of (i) a HER2/neu E75 peptide and/or a modified HER2/neu E75 peptide, and (ii) a cellular immunity induction promoter selected from a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenaline receptor antagonist, an adrenaline receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor, a Th2 cytokine inhibitor, and a combination of two or more of them; and
(47) a method for treating or preventing cancer in a subject, comprising administering to the subject a therapeutically effective amount of the cancer vaccine composition for mucosal administration according to any one of (1) to (42), the cancer vaccine film formulation for mucosal administration according to (43), the cancer vaccine liquid formulation for mucosal administration according to (44), or the cancer vaccine orally-disintegrating tablet for mucosal administration according to (45).

According to another aspect, the present invention also provides a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenaline receptor antagonist, an adrenaline receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor, a Th2 cytokine inhibitor, and a combination of two or more of them, as cellular immunity induction promoters for a HER2/neu E75 peptide and/or a modified HER2/neu E75 peptide. Therefore, the present invention also provides the following embodiment:
(48) A TLR ligahd, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenaline receptor antagonist, an adrenaline receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor, a Th2 cytokine inhibitor, or a combination of two or more of them, for the use as a cellular immunity induction promoter for mucosal administration of a HER2/neu E75 peptide and/or a modified HER2/neu E75 peptide.

The present invention also provides the following embodiments:
(49) A method of inducing cellular immunity, comprising mucosally administering to a subject (i) HER2/neu E75 peptide and/or a modified HER2/neu E75 peptide and (ii) a first cellular immunity induction promoter selected from TLR ligand, cyclic dinucleotide, helper peptide, immunomodulatory small molecule drug, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor, Th2 cytokine inhibitor and a combination of two or more kinds of them;
(50) TLR ligand, cyclic dinucleotide, helper peptide, immunomodulatory small molecule drug, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor, Th2 cytokine inhibitor, or a combination of two or more kinds of them, for use in promoting the induction of cellular immunity by the mucosal administration of HER2/neu E75 peptide and/or a modified HER2/neu E75 peptide;
(51) A combination of (i) HER2/neu E75 peptide and/or a modified HER2/neu E75 peptide and (ii) a first cellular immunity induction promoter selected from TLR ligand, cyclic dinucleotide, helper peptide, immunomodulatory small molecule drug, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor, Th2 cytokine inhibitor and a combination of two or more kinds of them, for use in inducing cellular immunity by the mucosal administration of HER2/neu E75 peptide and/or a modified HER2/neu E75 peptide;
(52) A combination of (i) HER2/neu E75 peptide and/or a modified HER2/neu E75 peptide and (ii) a cellular immunity induction promoter selected from TLR ligand, cyclic dinucleotide, helper peptide, immunomodulatory small molecule drug, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor, Th2 cytokine inhibitor and a combination of two or more kinds of them for use in treating or preventing a cancer, wherein the combination is mucosally administered to a subject; and
(53) Use of (i) HER2/neu E75 peptide and/or a modified HER2/neu E75 peptide and (ii) a cellular immunity induction promoter selected from TLR ligand, cyclic dinucleotide, helper peptide, immunomodulatory small molecule drug, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor, Th2 cytokine inhibitor, and a combination of two or more kinds of them, for the manufacture of a cancer vaccine composition for mucosal administration.

Since the cancer vaccine composition of the present invention can be mucosal administered (particularly, nasal administered and oral, including sublingual, mucosal administered), it has the following advantages: excellent compliance, for example, non-invasive administration, no pain, and release from fear of injection; patients can administer the cancer vaccine composition by himself/herself since the administration is simple; a risk of accidental infection due to needlestick injury by health care workers can be avoided; in the case of repetitive administration, the ambulatory frequency can be reduced, and this can contribute to the improvement in quality of life of the patient; and medical wastes which necessitate special disposition such as an injection needle are not generated. Further, there is also an advantage that efficacy of the cancer vaccine composition of the present invention is remarkably improved, as compared with the administration of a HER2/neu E75 peptide and/or a modified HER2/neu E75 peptide alone. Further, the cancer vaccine composition of the present invention also has an advantage that mucosal administration of the composition induce stronger cellular immunity as compared with injection administration.

### DETAILED DESCRIPTION OF THE INVENTION

First, terms used in the present specification will be defined so that the present invention can be more easily understood. Terms having no definition have the meaning which is normally understood by a person skilled in the art in the fields of, particularly, medicine, pharmacy, immunology, cell biology, biochemistry, polymer chemistry and the like, unless the context requires otherwise.

### I. Definitions

As used herein, the term "HER2/neu E75 peptide" means a peptide consisting of the sequence: Lys Ile Phe Gly Ser Leu Ala Phe Leu (SEQ ID NO: 1), which is derived from a product of oncogene HER2/neu (HER2 protein).

As used herein, the term "modified HER2/neu E75 peptide" means a peptide in which all or a part of amino acids of the HER2/neu E75 peptide are modified by substitution, modification or the like. The modified HER2/neu E75 peptides includes, for example,
(a) a peptide consisting of an amino acid sequence in which one to several, for example, 1, 2, 3, 4 or 5 amino acids are substituted, deleted or added in the amino acid sequence of HER2/neu E75 peptide; and
(b) a peptide consisting of an amino acid sequence in which all or a part of the amino acids, for example, one or more, for example, 1, 2, 3, 4, 5, 6, 7, 8 or 9 amino acids are modified in the amino acid sequence of HER2/neu E75 peptide.

Examples of "modification" of an amino acid which can be possessed by a modified HER2/neu E75 peptide include, but not limited to, acetylation; alkylation such as methylation; glycosylation, hydroxylation, carboxylation, aldehydation, phosphorylation, sulfonylation, formylation; aliphatic chain adding modification such as myristoylation, palmitoylation or stearoylation; octanoylation, esterification, amidation, deamidation; disulfide bond forming modification such as cystine modification, glutathione modification or thioglycolic acid modification; saccharification, ubiquitination, succinimide formation, glutamylation, and prenylation. The modified HER2/neu E75 peptide may contain substitution, deletion or addition of one or more amino acids and modification of one or more amino acids in combination.

The HER2/neu E75 peptide and/or the modified HER2/neu E75 peptide peptide can be in the free form, or any pharmacologically acceptable salt form, for example, a form of acid salts (acetic acid salt, TFA salt, hydrochloric acid salt, sulfuric acid salt, phosphoric acid salt, lactic acid salt, tartaric acid salt, maleic acid salt, fumaric acid salt, oxalic acid salt, hydrobromic acid salt, succinic acid salt, nitric acid salt, malic acid salt, citric acid salt, oleic acid salt, palmitic acid salt, propionic acid salt, formic acid salt, benzoic acid salt, picric acid salt, benzenesulfonic acid salt, dodecylsulfuric acid salt, methanesulfonic acid salt, p-toluenesulfonic acid salt, glutaric acid salt, a variety of amino acid salts etc.), metal salts (alkali metal salts (e.g. sodium salt, potassium salt), alkaline earth metal salts (e. g. calcium salt, magnesium salt), aluminum salt etc.), or amine salts (triethylamine salt, benzylamine salt, diethanolamine salt, t-butylamine salt, dicyclohexylamine salt, arginine salt, dimethylammonium salt, ammonium salt etc.). A preferable pharmacologically acceptable salt is an acetic acid salt or a TFA salt. The HER2/neu E75 peptide and/or the modified HER2/neu E75 peptide which has been synthesized or produced, isolated and purified by a well-known method can be used.

As used herein, the term "cellular immunity induction promoter" means any substance which can enhance the cellular immune response induced by an antigen which is administered together with the substance, as compared with the immune response induced by the antigen without the substance. The cellular immunity induction promoter may include substances specified in the present specification, though it is not limited by the action mechanism by which induction of the cellular immunity is promoted.

As used herein, the term "TLR ligand" means a ligand for a Toll-like receptor (TLR), and for example, ligands for TLR1 to TLR9 are included. Examples of the TLR ligand include a ligand of a heterodimer of TLR1 and TLR2 (TLR1/2 ligand), a ligand of a heterodimer of TLR2 and TLR6 (TLR2/6 ligand), a TLR2 and Dectin1 ligand, a TLR3 ligand, a TLR4 ligand, a TLR5 ligand, a TLR7 and/or TLR8 ligand, and a TLR9 ligand, all of which can be used as the cellular immunity induction promoter in the present invention. In a preferred aspect of the present invention, examples of the TLR ligand include a TLR1/2 ligand, a TLR3 ligand, a TLR4 ligand, and a TLR7 and/or TLR8 ligand.

As used herein, the term "TLR1/2 ligand" means a ligand of a heterodimer of a Toll-like receptor (TLR) 1 and a Toll-like receptor (TLR) 2, and includes, for example, a triacylated lipoprotein derived from a cell wall of a bacterium and a salt thereof, and these may be an extract, a product or a synthetic product, and are not limited to them.

In a preferred aspect of the present invention, the TLR1/2 ligand is Pam₃CSK₄. Pam₃CSK₄ has the formula:

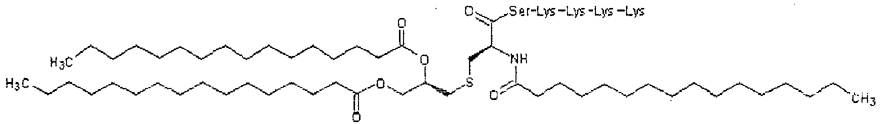

As used herein, the term "TLR2 and Dectin1 ligand" means a ligand of a Toll-like receptor (TLR) 2 and a β1,3-glucan receptor (Dectin1), and includes, for example, a β1,3-glucan derived from a cell wall of a fungus and a salt thereof, and these may be an extract, a product or a synthetic product, and are not limited to them. In a preferable aspect of the present invention, the TLR2 and Dectin1 ligand is Zymosan derived from a yeast cell wall.

As used herein, the term "TLR3 ligand" means a ligand of a Toll-like receptor (TLR) 3, and includes, for example, a double-stranded RNA (dsRNA) derived from a virus and a salt thereof, and these may be an extract, a product or a synthetic product, and are not limited to them. In a preferable aspect of the present invention, the TLR3 ligand is polyinosinic-polycytidylic acid (Poly(I:C)) which is a synthetic product and/or a salt thereof.

As used herein, the term "TLR4 ligand" means a ligand of a Toll-like receptor (TLR) 4, and includes, for example, a lipopolysaccharide (LPS) derived from a bacterium or a plant, particularly, a lipid A derivative, for example, monophosphoryl lipid A, a 3 deacylated monophosphoryl lipid A (3D-MPL), OM174, OM 294 DP or OM 197 MP-Ac DP and the like, alkyl glucosaminide phosphate (AGP), for example, AGP disclosed in WO 98/50399 or US 6303347, or a salt of AGP as disclosed in US 6764840, and a lipopolysaccharide derived from a Pantoea bacterium, a glucopyranosyl lipid, and sodium hyaluronate, but is not limited to them.

In a preferable aspect of the present invention, as the TLR4 ligand, lipopolysaccharides derived from genus *Acetobacter* (e.g. *Acetobacter aceti, Acetobacter xylinum, Acetobacter orientalis* etc.), genus *Zymomonas* (e.g. *Zymomonas mobilis* etc.), genus *Xanthomonas* (e.g. *Xanthomonas campestris* etc.), genus *Enterobacter* (e. g. *Enterobacter cloacae* etc.), and genus Pantoea (e.g. *Pantoea agglomerans* etc.) are preferable. Extracts derived from these lipopolysaccharides or purified lipopolysaccharides can be used as they are. In addition, for example, lipopolysaccharides (IP-PA1) derived from *Pantoea agglomerans* can be purchased from Funakoshi Corporation. In addition, in a preferable aspect of the present invention, the TLR4 ligand is a lipopolysaccharide derived from a *Pantoea* bacterium, glucopyranosyl lipid, and/or sodium hyaluronate.

As used herein, the term "TLR7 and/or TLR8 ligand" means a ligand of a Toll-like receptor (TLR) 7 and/or TLR8, and includes, for example, a single-stranded RNA, imiquimod, resiquimod (R848), TLR7-II and other compounds, for example, loxoribine and bropirimine, but is not limited to them.

In a preferable aspect of the present invention, the TLR7 and/or TLR8 ligand is imiquimod. Imiquimod is 1-(2-methylpropyl)-1H-imidazo[4,5-c]quinoline-4-amine of the formula: 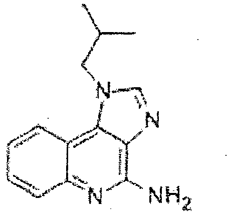 and, for example, the characteristics and a production process thereof are described in JP 7-505883 A (Patent Document 4).

In another preferred aspect, the TLR7 and/or TLR8 ligand is resiquimod. Resiquimod is 4-amino-2-(ethoxymethyl)-α,α-dimethyl-1H-imidazo[4,5-c]qui nolin-1-ethanol of the formula:

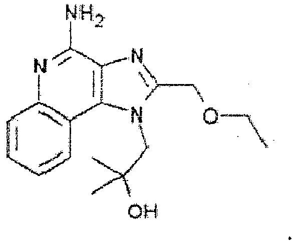

In another preferred aspect, the TLR7 and/or TLR8 ligand is TLR7-II. TLR7-II is represented by the formula:

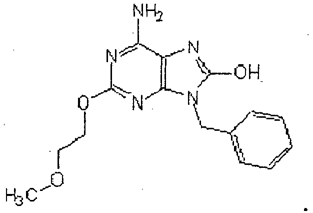

In another preferred aspect, the TLR7 and/or TLR8 ligand is bropirimine. Bropirimine is represented by the formula:

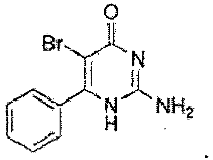

As used herein, the term "TLR9 ligand" means a ligand of a Toll-like receptor (TLR) 9, and includes, for example, ODN1826 and the like. The TLR9 ligand used in the present invention may be an extract, a product or a synthetic product, and is not limited to them. In a preferable aspect of the present invention, the TLR9 ligand is ODN1826.

ODN1826 is an oligodeoxynucleotide consisting of the following sequence (SEQ ID NO: 2):
5'-tccatgacgttcctgacgtt-3'

As used herein, the term "TLR2/6 ligand" means a ligand of a heterodimer of Toll-like receptor (TLR) 2 and a Toll-like receptor (TLR) 6, and includes, for example, a diacylated lipoprotein derived from a cell wall of mycoplasma and a salt thereof, and these may be an extract, a product or a synthetic product, and are not limited to them. In a preferable aspect of the present invention, the TLR2/6 ligand is Pam₂CSK₄, MALP-2 and/or FSL-1.

Pam₂CSK₄ is represented by the following formula:

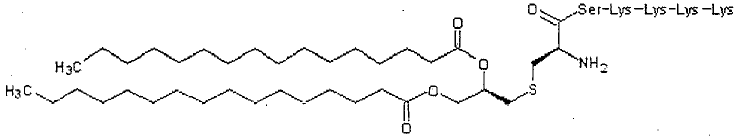

FSL-1 is represented by the following formula:

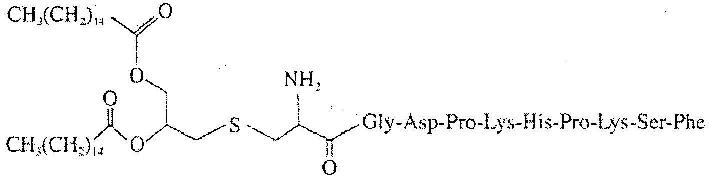

As used herein, the term "TLR5 ligand" means a ligand of a Toll-like receptor (TLR) 5, and includes, for example, flagellin and the like. The TLR5 ligand used in the present invention may be an extract, a product or a synthetic product, and is not limited to them. In a preferable aspect of the present invention, the TLR5 ligand is flagellin.

The Toll-like receptor (TLR) is a family of type I transmembrane proteins which initiates congenital immune response in which a specific cytokine, a specific chemokine and a growth factor participate, by in vivo activation thereof. All TLRs can activate a certain intracellular signal transmission molecule, for example, a nuclearity factor κB (NF-κB) and a mitogen-activated protein kinase (MAP kinase) or the like, while a specific population of a cytokine and a chemokine which are released seems to be inherent to each TLR. TLR1/2, TLR2/6, TLR4 and TLR5 are widely expressed in immune cells (dendritic cells, monocytes and the like) as well as general cells such as mucosal epithelial cells. It is known that these receptors recognize the constituent components of bacteria and promote the secretion of inflammatory cytokines (TNF-α, IL-1 and IL-6). In addition, it is known that TLR4 further promotes a production of type I interferons (IFNα and IFNβ). TLR3, 7, 8 and 9 include a subfamily of TLR which is present in an endosome fraction or a lysosome fraction of an immune cell (e.g. dendritic cell and monocyte). Specifically, TLR3 is expressed by a wide range of cells such as a dendritic cell and a fibroblast, TLR7 is expressed by a plasma cell-like dendritic cell, and is expressed by a monocyte to a lesser extent, TLR8 is expressed by a monocyte as well as a monocyte-derived dendritic cell and a myeloid dendritic cell, and TLR9 is expressed by a plasma cell-like dendritic cell. This subfamily mediates recognition of a microorganism nucleic acid (single-stranded RNA, double-stranded RNA, single-stranded DNA etc.). Agonists of TLR3, TLR7 and/or TLR8, and TLR9 stimulate production of a variety of inflammatory cytokines, for example, interleukin-6, interleukin-12, TNF-α, and interferon-γ. Such agonists also promote increase in expression of a costimulatory molecule, for example, CD40, CD80, and CD86, a major histocompatibility complex molecule, and a chemokine receptor. Type I interferons (IFNα and IFNβ) are produced by a cell upon activation with TLR7 and/or TLR8 agonists.

As used herein, the term "cyclic dinucleotide" means a molecule in which two OH groups of a sugar part of two nucleotides produce an ester for each same phosphoric acid molecule, and thereby nucleotides are cyclized, and an analog thereof, and includes, for example, cyclic di-AMP(c-di-AMP), cyclic di-GMP (c-di-GMP), c-dGpGp, c-dGpdGp, c-GpAp, c-GpCp, c-GpUp and the like, but is not limited to them. The cyclic dinucleotide activates a dendritic cell or a T cell. Further examples of the cyclic dinucleotide, use of them as an adjuvant, and a process for producing them are described in JP 2007-529531 A (Patent Document 5). In a preferable aspect of the present invention, the cyclic dinucleotide is cyclic di-GMP and/or cyclic di-AMP. The cyclic di-GMP has the formula: 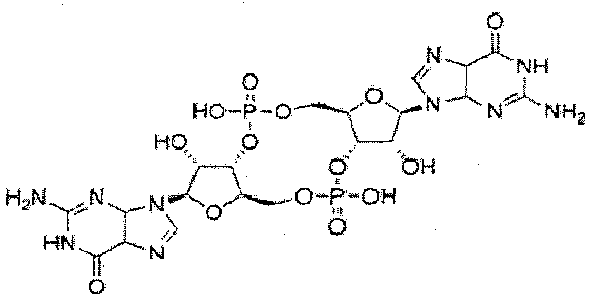 and a process for synthesizing it is described in Kawai et al., Nucleic Acids Research Suppl. 3: 103-4.

As used herein, the term "helper peptide" means any peptide which activates helper T cells, including, for example, a helper peptide derived from tuberculosis, a helper peptide derived from measles virus, a helper peptide derived from Hepatitis B virus, a helper peptide derived from hepatitis C virus, a helper peptide derived from Chlamydia trachomatis, a helper peptide derived from P. falciparum sporozoite, a helper peptide derived from keyhole limpet haemocyanin, a helper peptide derived from tetanus toxin, a helper peptide derived from pertussis toxin, a helper peptide derived from diphtheria toxin, a helper peptide derived from cancer cell (for example, an IMA-MMP-001 helper peptide, a CEA-006 helper peptide, an MMP-001 helper peptide, a TGFBI-004 helper peptide, an HER-2/neu (aa776-790) helper peptide, an AE36 helper peptide, an AE37 helper peptide, an MET-005 helper peptide, a BIR-002 helper peptide), and universal helper analogs (for example, PADRE). In a preferable aspect of the present invention, the helper peptide consists of 10 to 18 amino acids, preferably 12 to 16 amino acids, more preferably 13 to 15 amino acids. In a preferred aspect of the present invention, the helper peptide is Peptide-25 or PADRE. Peptide-25 is a peptide of 15 amino acids consisting of a sequence Phe Gln Asp Ala Tyr Asn Ala Gly His Asn Ala Val Phe (SEQ ID NO: 3), the sequence corresponds to the amino acid residue 240-254 of Ag85B, the major protein secreted by human-type tuberculosis (Mycobacterium tuberculosis). PADRE is a peptide of 13 amino acids consisting of a sequence D-Ala Lys cyclohexyl-Ala Val Ala Ala Trp Thr Leu Lys Ala Ala D-Ala (Herein referred as SEQ ID NO: 4).

Further, in the invention, instead of the helper peptide, or in combination of the helper peptide, any peptide modified by substitution or modification of all or some amino acids of the helper peptide (hereinafter referred as "modified helper peptide") can also be used.

The modified helper peptide includes, for example,
(a) a peptide consisting of an amino acid sequence in which one to several, for example, one, two, three, four or five amino acids are substituted, deleted or added, in an amino acid sequence of the original helper peptide; and
(b) a peptide consisting of an amino acid sequence in which all or a part of amino acids, for example, one or more, for example, one, two, three, four, five, six, seven, eight, nine, ten, eleven, twelve, thirteen, fourteen, or fifteen amino acids are modified, in an amino acid sequence of the original helper peptide.

Examples of the "modification" of an amino acid which can be possessed by the modified helper peptide include, but are not limited to, aliphatic chain addition modification such as acetylation, alkylation such as methylation, glycosylation, hydroxylation, carboxylation, aldehydization, phosphorylation, sulfonylation, formylation, addition of fatty acid such as myristoylation, palmitoylation and stearoylation, octanoylation, esterification, amidation, deamidation, disulfide bond formation modification such as cystine modification, glutathione modification and thioglycolic acid modification, glycation, ubiquitination, succinimide formation glutamylation, prenylation and the like. In addition, the modified helper peptide may contain a combination of substitution, deletion or addition of one or more amino acids, and modification of one or more amino acids.

As used herein, the term "cyclooxygenase inhibitor" means a substance which inhibits the function of cyclooxygenase (COX). This is also referred to as "COX inhibitor" hereinafter. As COX inhibitors, there are a COX inhibitor which selectively acts on particular cyclooxygenase (e.g. COX-1 or COX-2), and a COX inhibitor having no selectivity. Examples of COX inhibitors which can be used in the present invention include etodolac, loxoprofen, celecoxib, valdecoxib, parecoxib, lumiracoxib, meloxicam, tenoxicam, diclofenac, mefenamic acid, tolfenamic acid, flufenamic acid, meclofenamic acid, niflumic acid, benzydamine, indobufen, triflusal, tolmetin, fenoprofen, tiaprofenic acid, felbinac, nepafenac, amfenac, pravadoline, zaltoprofen, sulindac, nabumetone, diflunisal, piroxicam, ibuprofen, naproxen, fenoprofen, aspirin, methyl salicylate, salicylamide, salsalate, aloxiprin, tolmetin, indomethacin, proglumetacine, acemetacin, flurbiprofen, pranoprofen, acetaminophen, floctafenine, lornoxicam, tenoxicam, tiaprofenic acid, oxaprozin, ketoprofen, dexketoprofen, dexibuprofen, alminoprofen, ketorolac, mofezolac, phenylbutazone, oxyphenylbutazone, ketophenylbutazone, feprazone, phenbutazone, ethenzamide, tiaramide, tinoridine, epirizole, emorfazone and a derivative thereof, as well as a pharmacologically acceptable salt thereof. In a preferable aspect of the present invention, the COX inhibitor is etodolac and/or loxoprofen.

Loxoprofen is represented by the formula:

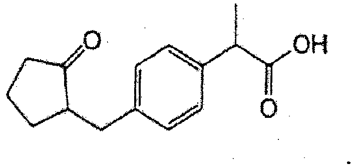

As used herein, the term "prostaglandin receptor antagonist" means a substance having the function of preventing prostaglandin from acting on a receptor, and includes, for example, an EP2 receptor antagonist, an EP4 receptor antagonist, a DP receptor antagonist, and an IP receptor antagonist.

As used herein, the term "EP2 receptor antagonist" means a substance having the function of preventing prostaglandin E2 from acting on an EP2 receptor. Examples of the EP2 receptor antagonist include AH6809 and a derivative thereof, as well as a pharmacologically acceptable salt thereof.

AH6809 is represented by the formula:

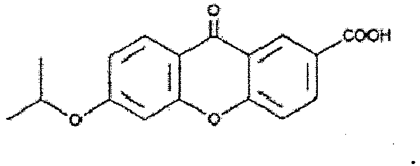

As used herein, the term "EP4 receptor antagonist" means a substance having the function of preventing prostaglandin E₂ from acting on an EP4 receptor. Examples of the EP4 receptor antagonist include GW627368X and a derivative thereof, as well as a pharmacologically acceptable salt thereof.

GW627368X is represented by the formula:

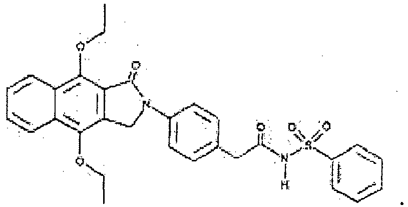

As used herein, the term "DP receptor antagonist" means a substance having the function of preventing prostaglandin D₂ from acting on a DP receptor. Examples of the DP receptor antagonist include S-5751, BWA868C and a derivative thereof, as well as a pharmacologically acceptable salt thereof.

BWA868C is represented by the formula:

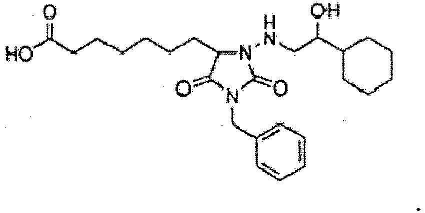

As used herein, the term "IP receptor antagonist" means a substance having the function of preventing prostaglandin I₂ from acting on an IP receptor. Examples of the IP receptor antagonist include RO1138452 and a derivative thereof, as well as a pharmacologically acceptable salt thereof.

RO1138452 is represented by the formula:

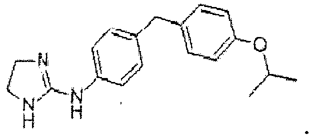

As used herein, the term "prostaglandin receptor agonist" means a substance having the function of acting on a prostaglandin receptor, and includes, for example, an EP3 receptor agonist.

As used herein, the term "EP3 receptor agonist" means a substance having the function of acting on an EP3 receptor. Examples of the EP3 receptor agonist include sulprostone, GR63799, cloprostenol, ONO-AE-248, carbacyclin, and a derivative thereof, as well as a pharmacologically acceptable salt thereof.

Sulprostone is represented by the formula:

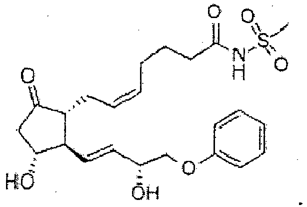

As used herein, the term "TSLP production inhibitor" means a substance having the function of inhibiting production of TSLP. Since a drug which inhibits NF-κB is thought to indirectly inhibit the production of TSLP, it is included in this category. Examples of the TSLP production inhibitor include naringenin, berberine, resveratrol, luteolin, apigenin, chrysoeriol, velutin, rutin, hesperidin, quercetin, daidzein, genistein, noscapine, diindolylmethane, xanthone, parthenolide and a derivative thereof, as well as a pharmacologically acceptable salt thereof.

Berberine is represented by the formula:

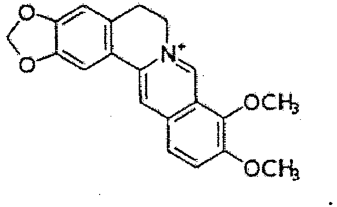

As used herein, the term "adenylate cyclase inhibitor" means a substance having the function of inhibiting the activity of adenylate cyclase. Examples of the adenylate cyclase inhibitor include 2',5'-dideoxyadenosine, niacin, insulin, and a derivative thereof, as well as a pharmacologically acceptable salt thereof.

2',5'-Dideoxyadenosine is represented by the formula:

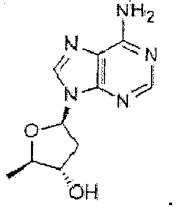

As used herein, the term "omega-3 fatty acid" refers to an unsaturated fatty acid having a carbon-carbon double bond at the ω-3 position. Examples of the omega-3 fatty acid include eicosapentaenoic acid, α-linolenic acid, docosahexaenoic acid, and a derivative thereof, as well as a pharmacologically acceptable salt thereof.

Eicosapentaenoic acid is represented by the formula:

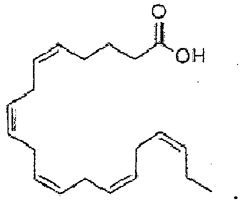

As used herein, the term "PPAR agonist" means a substance having the function of acting on a peroxisome proliferator-activated receptor, and includes, for example, a PPAR-α agonist, a PPAR-δ agonist, and a PPAR-γ agonist.

As used herein, the term "PPAR-α agonist" means a substance having the function of acting on an α type peroxisome proliferator-activated receptor. The term "PPAR-δ agonist" means a substance having the function of acting on a δ type peroxisome proliferator-activated receptor. The term "PPAR-γ agonist" means a substance having the function of acting on a γ type peroxisome proliferator-activated receptor. Examples of the PPAR-α agonist, and/or the PPAR-δ agonist, and/or the PPAR-γ agonist include clofibrate, fenofibrate, bezafibrate, ciprofibrate, etofibrate, telmisartan, oleyl ethanolamide, tetradecylthioacetic acid, troglitazone, pioglitazone, rosiglitazone, balaglitazone, rivoglitazone, ciglitazone, darglitazone, edaglitazone, netoglitazone, indeglitazar, tesaglitazar, muraglitazar, aleglitazar, and a derivative thereof, as well as a pharmacologically acceptable salt thereof.

Clofibrate is represented by the formula:

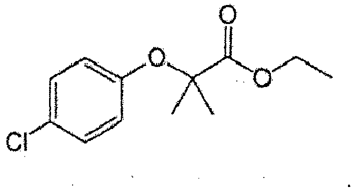

As used herein, the term "dopamine receptor antagonist" means a substance having the function of preventing dopamine from acting on a receptor, and includes, for example, a D1 receptor antagonist, and a D5 receptor antagonist.

As used herein, the term "D1 receptor antagonist" means a substance having the function of preventing dopamine from acting on a D1 receptor. Examples of the D1 receptor antagonist include benzazepine, fenoldopam, lorcaserin, SCH23390, SCH39166, LE300 and a derivative thereof, as well as a pharmacologically acceptable salt thereof.

Benzazepine is represented by the formula:

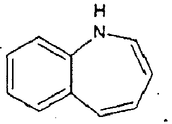

As used herein, the term "D5 receptor antagonist" means a substance having the function of preventing dopamine from acting on a D5 receptor. Examples of the D5 receptor antagonist include SCH39166 and a derivative thereof, as well as a pharmacologically acceptable salt thereof.

SCH39166 is represented by the formula:

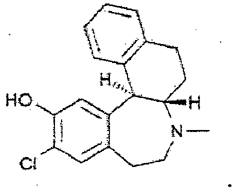

As used herein, the term "dopamine receptor agonist" means a substance having the function of acting on a dopamine receptor, and includes, for example, a D2 receptor agonist, a D3 receptor agonist, and a D4 receptor agonist.

As used herein, the term "D2 receptor agonist" means a substance having the function of acting on a D2 receptor. Examples of the D2 receptor agonist include cabergoline, bromocriptine, pergolide, ropinirole, talipexole, aripiprazole, lurasidone, and a derivative thereof, as well as a pharmacologically acceptable salt thereof.

Ropinirole is represented by the formula:

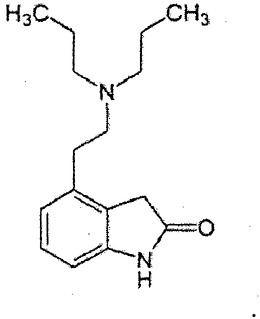

As used herein, the term "D3 receptor agonist" means a substance having the function of acting on a D3 receptor. Examples of the D3 receptor agonist include piribedil, rotigotine, PD1289077, OH-DPAT and a derivative thereof, as well as a pharmacologically acceptable salt thereof.

Rotigotine is represented by the formula:

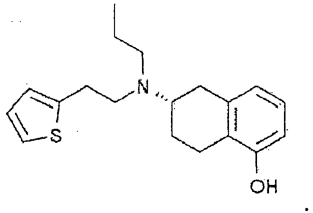

As used herein, the term "D4 receptor agonist" means a substance having the function of acting on a D4 receptor. Examples of the D4 receptor agonist include flibanserin, ABT724, PD168077, CP226269 and a derivative thereof, as well as a pharmacologically acceptable salt thereof.

Flibanserin is represented by the formula:

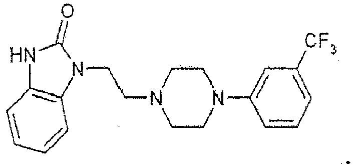

As used herein, the term "histamine receptor antagonist" means a substance having the function of preventing histamine from acting on a receptor, and includes, for example, a H1 receptor antagonist, and a H2 receptor antagonist.

As used herein, the term "H1 receptor antagonist" means a substance having the function of preventing histamine from acting on a H1 receptor. Examples of the H1 receptor antagonist include ketanserin, thonzylamine, mepyramine, tripelenamine, dimethindene, clemastine, bamipine, isothipendyl, chlorphenoxamine, dimetotiazine, chlorpromazine, hydroxyzine, opipramol, betahistine, cinnarizine, levocabastine, antazoline, diphenylpyraline, carbinoxamine, doxylamine, alimemazine, cyclizine, meclozine, levocetirizine, cyproheptadine, phenindamine, triprolidine, azatadine, astemizole, terfenadine, acrivastine, ebastine, desloratadine, rupatadine, bilastine, mizolastine, noberastine, rocastine, temelastine, bepotastine, diphenhydramine, chlorpheniramine, ketotifen, promethazine, cyproheptadine, epinastine, olopatadine, bepotastine, astemizole, emedastine, mequitazine, oxatomide, loratadine, fexofenadine, cetirizine, azelastine, and a derivative thereof, as well as a pharmacologically acceptable salt thereof.

Diphenhydramine is represented by the formula;

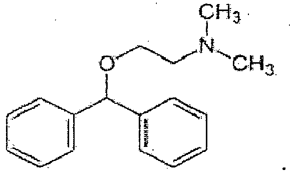

As used herein, the term "H2 receptor antagonist" means a substance having the function of preventing histamine from acting on a H2 receptor. Examples of the H2 receptor antagonist include cimetidine, ranitidine, famotidine, nizatidine, roxatidine, lafutidine, and a derivative thereof, as well as a pharmacologically acceptable salt thereof.

Famotidine is represented by the formula:

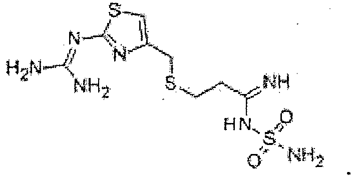

As used herein, the term "histamine receptor agonist" means a substance having the function of acting on a histamine receptor, and includes, for example, a H1 receptor agonist, a H3 receptor agonist, and a H4 receptor agonist.

As used herein, the term "H1 receptor agonist" means a substance having the function of acting on a H1 receptor. Examples of the H1 receptor agonist include 2-pyridylethylamine, 2-thiazolylethylamine and a derivative thereof, as well as a pharmacologically acceptable salt thereof.

2-Pyridylethylamine is represented by the formula:

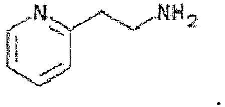

As used herein, the term "H3 receptor agonist" means a substance having the function of acting on a H3 receptor. Examples of the H3 receptor agonist include immethridine, imetit, immepip, α-methylhistamine, proxyfan, and a derivative thereof, as well as a pharmacologically acceptable salt thereof.

Proxyfan is represented by the formula:

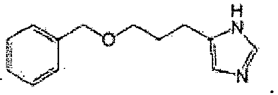

As used herein, the term "H4 receptor agonist" means a substance having the function of acting on a H4 receptor. Examples of the H4 receptor agonist include 4-methylhistamine, VUF8430, immepip and a derivative thereof, as well as a pharmacologically acceptable salt thereof.

4-Methylhistamine is represented by the formula:

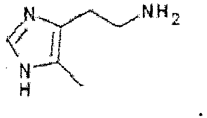

As used herein, the term "serotonin receptor antagonist" means a substance having the function of preventing serotonin from acting on a receptor, and includes, for example, a 5-HT2 receptor antagonist, a 5-HT4 receptor antagonist, a 5-HT6 receptor antagonist, and a 5-HT7 receptor antagonist.

As used herein, the term "5-HT2 receptor antagonist" means a substance having the function of preventing serotonin from acting on a 5-HT2 receptor. Examples of the 5-HT2 receptor antagonist include pizotifen, risperidone, olanzapine, quetiapine, aripiprazole, blonanserin, clozapine, paliperidone, ritanserin, yohimbine, mesulergine, agomelatine, cyclobenzaprine, sarpogrelate, methysergide, ketanserin, and a derivative thereof, as well as a pharmacologically acceptable salt thereof.

Olanzapine is represented by the formula:

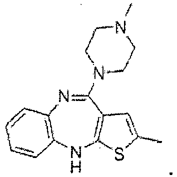

As used herein, the term "5-HT4 receptor antagonist" means a substance having the function of preventing serotonin from acting on a 5-HT4 receptor. Examples of the 5-HT4 receptor antagonist include piboserod, GR113808, GR125487, RS39604, SB204070 and a derivative thereof, as well as a pharmacologically acceptable salt thereof.

Piboserod is represented by the formula:

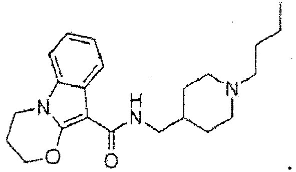

As used herein, the term "5-HT6 receptor antagonist" means a substance having the function of preventing serotonin from acting on a 5-HT6 receptor. Examples of the 5-HT6 receptor antagonist include cerlapirdine, clozapine and a derivative thereof, as well as a pharmacologically acceptable salt thereof.

Cerlapirdine is represented by the formula:

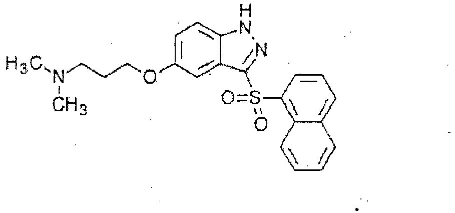

As used herein, the term "5-HT7 receptor antagonist" means a substance having the function of preventing serotonin from acting on a 5-HT7 receptor. Examples of the 5-HT7 receptor antagonist include lurasidone, metergoline, and a derivative thereof, as well as a pharmacologically acceptable salt thereof.

Metergoline is represented by the formula:

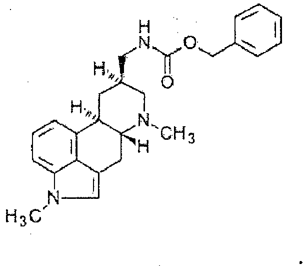

As used herein, the term "serotonin receptor agonist" means a substance having the function of acting on a serotonin receptor, and includes, for example, a 5-HT1 receptor agonist, and a 5-HT2 receptor agonist.

As used herein, the term "5-HT1 receptor agonist" means a substance having the function of acting on a 5-HT1 receptor. Examples of the 5-HT1 receptor agonist include piclozotan, tandospirone, sumatriptan, zolmitriptan, eletriptan, rizatriptan, naratriptan, almotriptan, frovatriptan, avitriptan, ergotamine, ergot alkaloid, and a derivative thereof, as well as a pharmacologically acceptable salt thereof.

Zolmitriptan is represented by the formula:

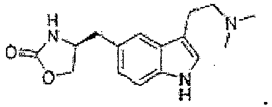

As used herein, the term "5-HT2 receptor agonist" means a substance having the function of acting on a 5-HT2 receptor. Examples of the 5-HT2 receptor agonist include α-methyl-5-HT, agomelatine, norfenfluramine, meta-chlorophenylpiperazine and a derivative thereof, as well as a pharmacologically acceptable salt thereof.

Agomelatine is represented by the formula:

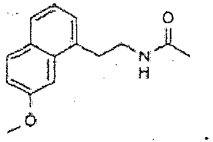

As used herein, the term "vasopressin receptor antagonist" means a substance having the function of preventing vasopressin from acting on a receptor, and includes, for example, a V2 receptor antagonist.

As used herein, the term "V2 receptor antagonist" means a substance having the function of preventing vasopressin from acting on a V2 receptor. Examples of the V2 receptor antagonist include tolvaptan, mozavaptan, conivaptan, lixivaptan, and a derivative thereof, as well as a pharmacologically acceptable salt thereof.

Mozavaptan is represented by the formula:

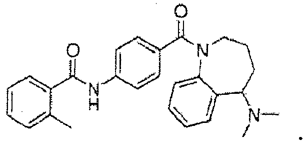

As used herein, the term "vasopressin receptor agonist" means a substance having the function of acting on a vasopressin receptor, and includes, for example, a V1 receptor agonist.

As used herein, the term "V1 receptor agonist" means a substance having the function of acting on a V1 receptor. Examples of the V1 receptor agonist include vasopressin, felypressin, desmopressin, lypressin, terlipressin, ornipressin, argipressin, and a derivative thereof, as well as a pharmacologically acceptable salt thereof.

Desmopressin is represented by the formula:

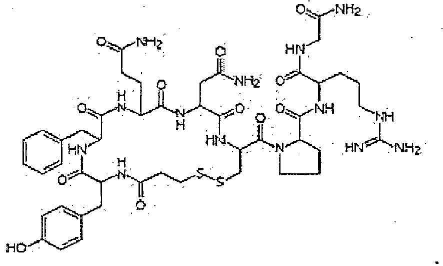

As used herein, the term "muscarine receptor antagonist" means a substance having the function of preventing acetylcholine from acting on a muscarine receptor, and includes, for example, a M1 receptor antagonist, a M3 receptor antagonist, and a M5 receptor antagonist.

As used herein, the term "M1 receptor antagonist" means a substance having the function of preventing acetylcholine from acting on a M1 receptor. The term "M3 receptor antagonist" means a substance having the function of preventing acetylcholine from acting on a M3 receptor. The term "M5 receptor antagonist" means a substance having the function of preventing acetylcholine from acting on a M5 receptor. Examples of the M1 receptor antagonist, and/or the M3 receptor antagonist, and/or the M5 receptor antagonist include pirenzepine, atropine, trimebutine, piperidolate, oxybutynin, tropicamide, propiverine, tolterodine, solifenacin, darifenacin, imidafenacin, oxyphencyclimine, tiotropium bromide, esoxybutynin, tiquizium, and a derivative thereof, as well as a pharmacologically acceptable salt thereof.

Oxybutynin is represented by the formula:

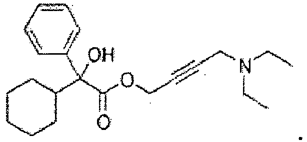

As used herein, the term "muscarine receptor agonist" means a substance having the function of acting on a muscarine receptor, and includes, for example, a M1 receptor agonist, a M2 receptor agonist, a M3 receptor agonist, a M4 receptor agonist, and a M5 receptor agonist.

As used herein, the term "M1 receptor agonist" means a substance having the function of acting on a M1 receptor. The term "M2 receptor agonist" means a substance having the function of acting on a M2 receptor. The term "M3 receptor agonist" means a substance having the function of acting on a M3 receptor. The term "M4 receptor agonist" means a substance having the function of acting on a M4 receptor. The term "M5 receptor agonist" means a substance having the function of acting on a M5 receptor. Examples of the M1 receptor agonist, and/or the M2 receptor agonist, and/or the M3 receptor agonist, and/or the M4 receptor agonist, and/or the M5 receptor agonist include acetylcholine, aceclidine, alvameline, talsaclidine, xanomeline, pilocarpine, cevimeline, bethanechol, mazaticol, muscarine, and a derivative thereof, as well as a pharmacologically acceptable salt thereof.

Bethanechol is represented by the formula:

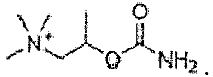

As used herein, the term "adrenalin receptor antagonist" means a substance having the function of preventing adrenalin from acting on a receptor, and includes, for example, an α1 receptor antagonist, a β1 receptor antagonist, a β2 receptor antagonist, and a β3 receptor antagonist.

As used herein, the term "α1 receptor antagonist" means a substance having the function of preventing adrenalin from acting on an α1 receptor. Examples of the α1 receptor antagonist include prazosin, doxazosin, bunazosin, trimazosin, alfuzosin, silodosin, terazosin, tamusulosin, and a derivative thereof, as well as a pharmacologically acceptable salt thereof.

Tamsulosin is represented by the formula:

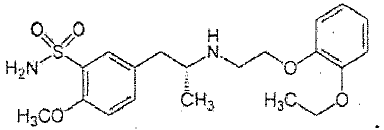

As used herein, the term "β1 receptor antagonist" means a substance having the function of preventing adrenalin from acting on a β1 receptor. The term "β2 receptor antagonist" means a substance having the function of preventing adrenalin from acting on a β2 receptor. The term "β3 receptor antagonist" means a substance having the function of preventing adrenalin from acting on a β3 receptor. Examples of the β1 receptor antagonist, and/or the β2 receptor antagonist, and/or the β3 receptor antagonist include bopindolol, pindolol, timolol, dichloroisoprenaline, alprenolol, carteolol, indenolol, bunitrolol, penbutolol, propranolol, nadolol, nipradilol, tilisolol, acebutolol, celiprolol, metoprolol, atenolol, bisoprolol, betaxolol, practolol, bevantolol, butoxamine, carvedilol, amosulalol, arotinolol, labetalol, and a derivative thereof, as well as a pharmacologically acceptable salt thereof.

Propranolol is represented by the formula:

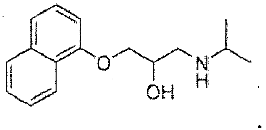

As used herein, the term "angiotensin receptor agonist" means a substance having the function of acting on an angiotensin receptor, and includes, for example, an AT2 receptor agonist.

As used herein, the term "adrenalin receptor agonist" means a substance having the function of acting on an adrenalin receptor, and includes, for example, an α1 receptor agonist, and an α2 receptor agonist.

As used herein, the term "α1 receptor agonist" means a substance having the function of acting on an α1 receptor. The term "α2 receptor agonist" means a substance having the function of acting on an α2 receptor. Examples of the α1 receptor agonist, and/or the α2 receptor agonist include norepinephrine, norfenefrine, etilefrine, naphazoline, phenylephrine, midodrine, methoxamine, oxedrine, metaraminol, arbutamine, ephedrine, oxymetazoline, tetryzoline, xylometazoline, tramazoline, pseudoephedrine, dipivefrine, amidephrine, methylephedrine, rilmenidine, brimonidine, medetomidine, xylazine, tizanidine, guanfacine, methyldopa, guanabenz, and a derivative thereof, as well as a pharmacologically acceptable salt thereof.

Xylazine is represented by the formula:

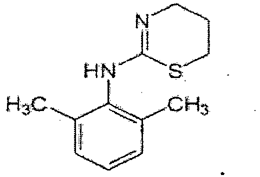

As used herein, the term "angiotensin receptor agonist" means a substance having the function of acting on an angiotensin receptor, and includes, for example, an AT2 receptor agonist.

As used herein, the term "AT2 receptor agonist" means a substance having the function of acting on an AT2 receptor. Examples of the AT2 receptor agonist include novokinin, angiotensin and a derivative thereof, as well as a pharmacologically acceptable salt thereof.

Angiotensin is represented by the formula:

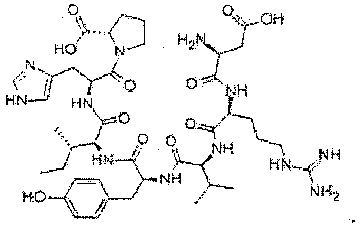

As used herein, the term "GABA receptor agonist" means a substance having the function of acting on a GABA receptor, and includes, for example, a GABA_{B} receptor agonist.

As used herein, the term "GABA_{B} receptor agonist" means a substance having the function of acting on a GABA_{B} receptor. Examples of the GABA_{B} receptor agonist include baclofen, γ-aminobutyric acid, arbaclofen and a derivative thereof, as well as a pharmacologically acceptable salt thereof.

Baclofen is represented by the formula:

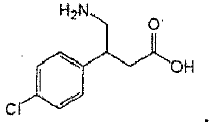

As used herein, the term "thrombin receptor antagonist" means a substance having the function of preventing thrombin from acting on a receptor, and includes, for example, a PAR-1 receptor antagonist.

As used herein, the term "PAR-1 receptor antagonist" means a substance having the function of preventing thrombin from acting on a PAR-1 receptor. Examples of the PAR-1 receptor antagonist include vorapaxar, atopaxar, FR171113, RWJ56110, dabigatran, dabigatran etexilate, melagatran, ximelagatran, hirudin, hirulog, argatroban and a derivative thereof, as well as a pharmacologically acceptable salt thereof.

Vorapaxar is represented by the formula:

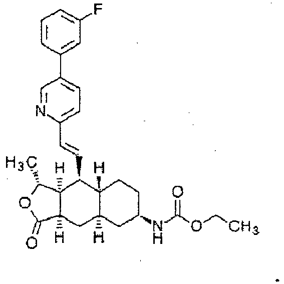

As used herein, the term "thrombin receptor agonist" means a substance having the function of acting on a thrombin receptor, and includes, for example, a PAR-1 receptor agonist.

As used herein, the term "PAR-1 receptor agonist" means a substance having the function of acting on a PAR-1 receptor. Examples of the PAR-1 receptor agonist include TRAP-6, TRAP-14, NAT6-NH₂ and a derivative thereof, as well as a pharmacologically acceptable salt thereof.

TRAP-6 is represented by the formula:

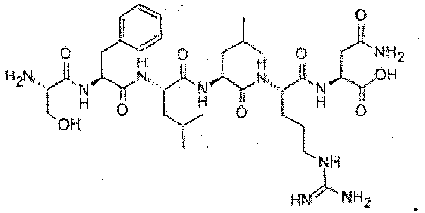

As used herein, the term "opioid receptor agonist" means a substance having the function of acting on an opioid receptor. Examples of the opioid receptor agonist include trimebutine, alvimopan, morphine, oxycodone, dihydrocodeine, diamorphine, pethidine, pentazocine, buprenorphine, butorphanol, nalbuphine, tilidine, dezocine, meptazinol, tapentadol, naltrexone, methadone, ethylmorphine, hydrocodone, acetyldihydrocodeine, nalorphine, loperamide, remoxipride, opipramol, and a derivative thereof, as well as a pharmacologically acceptable salt thereof.

Buprenorphine is represented by the formula:

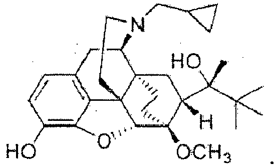

As used herein, the term "leukotriene receptor antagonist" means a substance having the function of preventing leukotriene from acting on a receptor, and includes, for example, a CysLT1 receptor antagonist, and a CysLT2 receptor antagonist.

As used herein, the term "CysLT1 receptor antagonist" means a substance having the function of preventing leukotriene from acting on a CysLT1 receptor. The term "CysLT2 receptor antagonist" means a substance having the function of preventing leukotriene from acting on a CysLT2 receptor. Examples of the CysLT1 receptor antagonist, and/or the CysLT2 receptor antagonist include montelukast, zafirlukast, pranlukast, and a derivative thereof, as well as a pharmacologically acceptable salt thereof. Examples of the pharmacologically acceptable salt of montelukast include montelukast sodium and the like.

Montelukast sodium is represented by the formula:

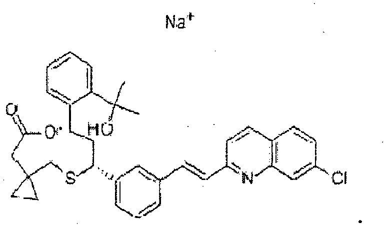

As used herein, the term "leukotriene receptor agonist" means a substance having the function of acting on a leukotriene receptor, and includes, for example, a BLT receptor agonist.

As used herein, the term "BLT receptor agonist" means a substance having the function of acting on a BLT receptor. Examples of the BLT receptor agonist include leukotriene B4, CAY10583 and a derivative thereof, as well as a pharmacologically acceptable salt thereof.

Leukotriene B4 is represented by the formula:

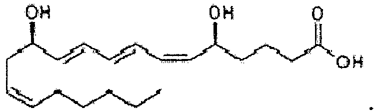

As used herein, the term "ADP receptor agonist" means a substance having the function of acting on an ADP receptor. Examples of the ADP receptor agonist include adenosine diphosphate, and a derivative thereof, as well as a pharmacologically acceptable salt thereof.

Adenosine diphosphate is represented by the formula:

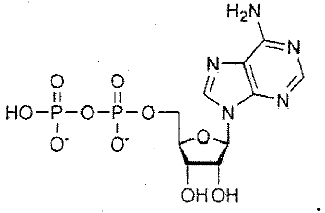

As used herein, the term "melatonin receptor agonist" means a substance having the function of acting on a melatonin receptor. Examples of the melatonin receptor agonist include melatonin, perlapine, tasimelteon, and a derivative thereof, as well as a pharmacologically acceptable salt thereof.

Melatonin is represented by the formula:

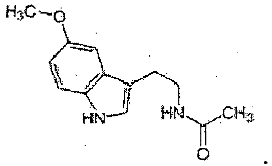

As used herein, the term "somatostatin receptor agonist" means a substance having the function of acting on a somatostatin receptor. Examples of the somatostatin receptor agonist include somatostatin, somatostatin-14, octreotide, and a derivative thereof, as well as a pharmacologically acceptable salt thereof.

Octreotide is represented by the formula:

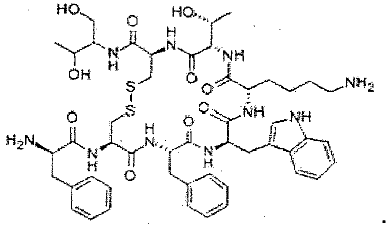

As used herein, the term "cannabinoid receptor agonist" means a substance having the function of acting on a cannabinoid receptor. Examples of the cannabinoid receptor agonist include dronabinol, nabilone, levonantradol, otenabant, GW833972A, GW405833, and a derivative thereof, as well as a pharmacologically acceptable salt thereof.

Dronabinol is represented by the formula:

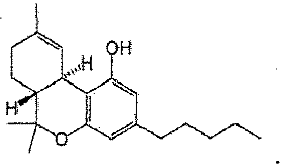

As used herein, the term "sphingosine-1 phosphate receptor agonist" means a substance having the function of acting on a sphingosine-1 phosphate receptor. Examples of the sphingosine-1 phosphate receptor agonist include fingolimod, ponesimod, RPC-1063, ONO-4641, SEW2871, sphingosine-1 phosphate and a derivative thereof, as well as a pharmacologically acceptable salt thereof.

Fingolimod is represented by the formula:

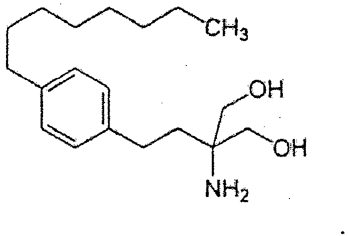

As used herein, the term "metabotropic glutamate receptor agonist" means a substance having the function of acting on a metabotropic glutamate receptor, and includes, for example, an mGluR2 receptor agonist, an mGluR3 receptor agonist, an mGluR4 receptor agonist, an mGluR6 receptor agonist, an mGluR7 receptor agonist, and an mGluR8 receptor agonist.

As used herein, the term "mGluR2 receptor agonist" means a substance having the function of acting on an mGluR2 receptor. The term "mGluR3 receptor agonist" means a substance having the function of acting on an mGluR3 receptor. The term "mGluR4 receptor agonist" means a substance having the function of acting on an mGluR4 receptor. The term "mGluR6 receptor agonist" means a substance having the function of acting on an mGluR6 receptor. The term "mGluR7 receptor agonist" means a substance having the function of acting on an mGluR7 receptor. The term "mGluR8 receptor agonist" means a substance having the function of acting on an mGluR8 receptor. Examples of the mGluR2 receptor agonist, and/or the mGluR3 receptor agonist, and/or the mGluR4 receptor agonist, and/or the mGluR6 receptor agonist, and/or the mGluR7 receptor agonist, and/or the mGluR8 receptor agonist include VU0361737, VU0155041, biphenylindanone A, PBDA, L-AP4, and a derivative thereof, as well as a pharmacologically acceptable salt thereof.

VU0361737 is represented by the formula:

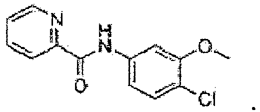

As used herein, the term "phospholipase A2 inhibitor" means a substance having the function of inhibiting the activity of phospholipase A2. Examples of the phospholipase A2 inhibitor include glycyrrhizic acid, glycyrrhetic acid, and a derivative thereof, as well as a pharmacologically acceptable salt thereof.

Glycyrrhetic acid is represented by the formula:

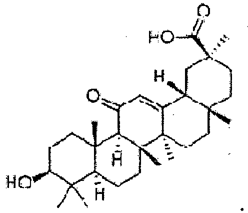

As used herein, the term "TGF-β production inhibitor" means a substance having the function of inhibiting production of TGF-β. Examples of the TGF-β production inhibitor include pirfenidone, tranilast, and a derivative thereof, as well as a pharmacologically acceptable salt thereof.

Pirfenidone is represented by the formula:

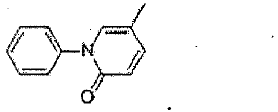

As used herein, the term "Th2 cytokine inhibitor" means a substance having the function of inhibiting production of a Th2 cytokine such as IL-4 and IL-5. Examples of the Th2 cytokine inhibitor include suplatast and a derivative thereof, as well as a pharmacologically acceptable salt thereof. Examples of the pharmacologically acceptable salt of suplatast include suplatast tosylate. In a preferable aspect of the present invention, the Th2 cytokine inhibitor is suplatast tosylate.

Suplatast tosylate is represented by the formula:

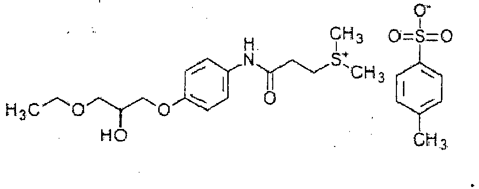

As used herein, the "pharmacologically acceptable acid" as a second cellular immunity induction promoter that can be contained in the composition of the present invention means an acid which has no harmful effect on a subject to which the composition is administered, and does not impair the pharmacological activity of the ingredients in the composition. In a preferable aspect of the present invention, the pharmacologically acceptable acid is an organic acid, more preferably an organic compound containing carboxyl group or an organic compound containing sulfo group, more preferably saturated or unsaturated straight or branched fatty acid in which the saturated straight chain part has 8 to 20 carbon atoms, lactic acid, malic acid, salicylic acid, maleic acid, citric acid, or an organic compound containing sulfo group, more preferably saturated or unsaturated straight or branched fatty acid in which the saturated straight chain part has 8 to 16 carbon atoms, lactic acid, malic acid, salicylic acid, maleic acid, citric acid, an organic compound containing sulfo group, further preferably fatty acid selected from the group consisting of decanoic acid, lauric acid, myristic acid, isostearic acid, palmitic acid, stearic acid and oleic acid, or lactic acid, salicylic acid, citric acid or methanesulfonic acid.

As used herein, a "pharmacologically acceptable salt" which can be contained in the composition of the present invention means a salt which has no harmful effect on an the subject to be administered with the composition, and does not impair the pharmacological activity of the ingredients in the composition. Examples of pharmacologically acceptable salts include inorganic acid salts (e.g. hydrochloric acid salt and phosphoric acid salt), organic acid salts (e. g. acetic acid salt, phthalic acid salt, and TFA salt), metal salts (alkali metal salts (e.g. sodium salt and potassium salt), alkaline earth metal salts (e.g. calcium salt and magnesium salt), aluminum salt etc.), and amine salts (triethylamine salt, benzylamine salt, diethanolamine salt, t-butylamine salt, dicyclohexylamine salt, arginine salt, dimethylammonium salt, ammonium salt etc.), but are not limited to them.

As used herein, the term "immunomodulatory small molecule drug" means a substance which activates or suppresses immune cells such as a T cell, a NK cell, a macrophage and the like, and which does not correspond to any of the aforementioned TLR ligand, cyclic dinucleotide, helper peptide, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-β production inhibitor, and Th2 cytokine inhibitor. Examples of the immunomodulatory small molecule drug include bestatin, pidotimod, levamisole, golotimod, forphenicinol, and a derivative thereof, as well as a pharmacologically acceptable salt thereof. Examples of the pharmacologically acceptable salt of levamisole include levamisole hydrochloride.

Bestatin is represented by the formula:

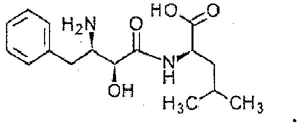

Pidotimod is represented by the formula:

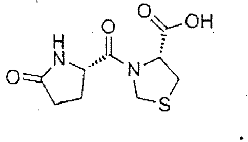

Levamisole hydrochloride is represented by the formula:

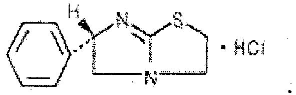

In the present invention, the immunomodulatory small molecule drug is usually a compound having a molecular weight of less than 1000, preferably less than 500. In a preferable aspect of the present invention, the immunomodulatory small molecule drug is one or more compounds selected from the group consisting of bestatin, pidotimod and levamisole hydrochloride.

As above described, the inventors have found that among various cellular immunity induction promoters, a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenaline receptor antagonist, an adrenaline receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor, and a Th2 cytokine inhibitor are particularly suitable for the mucosal administration of a HER2/neu E75 peptide and/or a modified HER2/neu E75 peptide. Therefore, in an aspect of the present invention, the cellular immunity induction promoter of the present invention is one or more substances selected from them. In further aspect, such a substance is a first cellular immunity induction promoter. In a particularly preferred aspect of the present invention, the cellular immunity induction promoter is a combination of one or more substances selected from a TLR ligand, a cyclic dinucleotide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenaline receptor antagonist, an adrenaline receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor and a Th2 cytokine inhibitor, and a helper peptide. The induction of cellular immunity can be determined quantitatively by a variety of known methods. Any of those known method, for example, the ELISPOT method described in Examples may be used in this application.

As used herein, non-invasive administration means administration without actively giving physical irritation and/or chemical irritation, preferably without giving physical irritation (e.g. without giving irritation by mucosal peeling treatment, mucosal damaging treatment, and mucosal perforation treatment) to a mucosa.

As used herein, the term "cancer" means cancer associated with abnormal expression, for example, overexpression of the HER2/neu gene. Examples of cancer may include adenocarcinoma. Examples of the adenocarcinoma associated with abnormal expression of a HER2/neu gene include, but are not limited to, breast cancer and prostate cancer.

As used herein, the term "abnormal expression of a gene" means that the expression level of the gene in a cell is increased or decreased remarkably, for example, by 2 times or more such as by 4 times or more, as compared with the other cells in the same tissue. The term "overexpression" means that the abnormal expression is an increase in the expression level. The expression level of a gene can be easily measured using any method well-known in the art.

As used herein, the term "subject" means any animal having the HER2/neu gene, immune response of which can be induced by the transdermal administration of a cancer vaccine composition for transdermal administration at a practical stage. Typically, the subject may be a mammal such as human, mouse, rat, dog, cat, rabbit, horse, cow, sheep, pig, goat, monkey, and chimpanzee. A particularly preferable subject is human.

II. Cancer vaccine composition for mucosal administration

The usefulness of the HER2/neu E75 peptide as a cancer vaccine has been already apparent (Non-Patent Document 3, Non-Patent Document 4).

As used herein, the term, composition for "mucosal administration" means any formulation or preparation which is usually used for mucosal administration, for example, sublingual, transnasal, buccal, rectal or vaginal administration. The composition may be, for example, a semisolid formulation such as a gel formulation (jelly formulation), a cream formulation, an ointment, and a plaster formulation; a liquid formulation; a solid formulation such as a powdered formulation, a fine granule formulation, a granule formulation, a film formulation, a tablet and an orally-disintegrating tablet; a mucosal spray formulation such as an aerosol formulation; and an inhalant formulation. Grouping, definition, a nature, a production process and the like of these formulations are well known in the art. For example, see the Japanese Pharmacopoeia, 16^{th} Edition.

For example, as a solvent for liquid formulation, an appropriate amount of water or solvent such as ethanol, glycerin or propylene glycol can be used, and a liquid formulation can be prepared by dispersing or dissolving ingredients in the solvent.

As the base for gel formulation (jelly formulation), for example, a carboxyvinyl polymer as a hydrogel base, a gel base, a fat-free ointment, polyvinylpyrrolidone, polyvinyl alcohol, sodium polyacrylate, carboxymethyl cellulose, starch, xanthan gum, karaya gum, sodium alginate, methyl cellulose, hydroxypropyl cellulose, hydroxypropyl methyl cellulose phthalate (HPMCP), cellulose acetate phthalate (CAP), carboxymethyl ethyl cellulose (CMEC), ethyl cellulose, hydroxyethyl cellulose, hydroxypropyl methyl cellulose, a carboxyvinyl polymer, tragacanth gum, gum arabic, tara gum, tamarind seed gum, psyllium seed gum, agar, gellan gum, glucomannan, locust bean gum, guar gum, carrageenan, dextrin, dextran, amylose, carboxymethyl cellulose potassium, carboxymethyl cellulose sodium, carboxymethyl cellulose calcium, pullulan, chitosan, carboxymethyl starch sodium, Plantago testa, galactomannan, eudragit, casein, alginic acid alkyl esters, gelatin, and polyethylene glycol can be used. These bases can be dissolved in a solvent to prepare a gel formulation having fluidity or a gel formulation having formability. The solvent is preferably water, while glycerin or propylene glycol can also be used.

Examples of the base for cream formulation include water/oil type bases such as hydrophilic ointment and vanishing cream; and oil/water type bases such as hydrophilic petrolatum, purified lanolin, Aquahole, Eucerin, Neocerin, hydrated lanolin, cold cream, and hydrophilic plastibase. A cream formulation can be prepared by adding these bases into oil and fat-based solvents or water, and stirring the mixture at high speed using a homogenizer or the like.

Examples of the base for film formulation include polyvinylpyrrolidone, polyvinyl alcohol, sodium polyacrylate, carboxymethyl cellulose, starch, xanthan gum, karaya gum, sodium alginate, methyl cellulose, a carboxyvinyl polymer, agar, hydroxypropyl cellulose, hydroxypropyl methyl cellulose phthalate (HPMCP), cellulose acetate phthalate (CAP), carboxymethyl ethyl cellulose (CMEC), ethyl cellulose, hydroxyethyl cellulose, hydroxypropyl methyl cellulose, a carboxyvinyl polymer, tragacanth gum, gum arabic, locust bean gum, guar gum, carrageenan, dextrin, dextran, amylose, carboxymethyl cellulose potassium, carboxymethyl cellulose sodium, carboxymethyl cellulose calcium, pullulan, chitosan, carboxymethyl starch sodium, Plantago testa, galactomannan, aminoalkyl methacrylate copolymer E, aminoalkyl methacrylate copolymer RS, methacrylic acid copolymer L, methacrylic acid copolymer LD, methacrylic acid copolymer S, methyl acrylate-methacrylic acid-methyl methacrylate copolymer, ethyl acrylate-methyl methacrylate copolymer, polyvinyl acetal diethyl aminoacetate, casein, and alginic acid alkyl ester. A film formulation can be prepared by dissolving these bases in water or an organic polar solvent such as ethanol, applying the solution into a thin film, and drying the solution. In one preferred aspect, the vaccine composition for mucosal administration of the present invention is in the form of a film formulation.

For example, the powder formulation, the fine granule formulation, the granule formulation and the tablet can be prepared by mixing excipients such as lactose, corn starch and crystalline cellulose; binders such as hydroxypropyl cellulose and gum arabic; and an appropriate amount of a solvent such as water or ethanol, and then subjecting the mixture to granulation, drying, tableting or combination thereof. If necessary, a lubricant such as magnesium stearate, and a coating agent such as hydroxypropyl cellulose or sucrose can also be added.

Examples of the base for an orally-disintegrating tablet (freeze-dried type) include gelatin and polysaccharides such as pullulan. Further, mannitol, trehalose, sorbitol, glycine and the like can also be used as a forming agent. An orally-disintegrating tablet (freeze-dried type) can be prepared by dissolving these additives in water, dispensing the solution, and freeze-drying the solution. In one preferred aspect, the vaccine composition for mucosal administration of the present invention is in the form of an orally-disintegrating tablet.

For example, the aerosol formulation includes, as its content, a liquid formulation, a gel formulation having high fluidity, a cream formulation, and a fine powder such as a powder formulation. These can be administered to a site of administration such as oral mucosa and nasal mucosa with high efficiency, by dispersing solid or liquid microparticles in a gas using a spraying device.

The amount of the HER2/neu E75 peptide and/or modified HER2/neu E75 peptide and the cellular immunity induction promoter in the composition of the present invention is not particularly limited. In an aspect, the composition of the present invention contains the HER2/neu E75 peptide and/or modified HER2/neu E75 peptide preferably in an amount of 0.01% to 40% by weight, and more preferably 0.1% to 30% by weight, relative to the total weight of the composition. In an aspect, the composition of the present invention contains the cellular immunity induction promoter preferably in an amount of 0.001% to 30% by weight, and more preferably 0.01% to 20% by weight, relative to the total weight of the composition.

Further, the composition of the present invention may contain an additive, if necessary. The additive is selected from, for example, isotonizing agents, antiseptics/germicides, antioxidants, resolvents, solubilizers, suspending agents, fillers, pH adjusting agents, stabilizers, absorption promoters, release rate controlling agents, colorants, plasticizers, adhesives and combinations of two or more of them, depending on the compatibility with the main ingredient of the base, the HER2/neu E75 peptide and/or modified HER2/neu E75 peptide and the cellular immunity induction promoter, the intended administration regimen and the like.

The therapeutically effective amount of the HER2/neu E75 peptide and/or the modified HER2/neu E75 peptide may widely vary depending on severity of the disease, age and relative health of the subject and other known factors. In general, satisfactory result may be obtained at a one day dose of about 0.1 µg to 1 g/kg body weight. The cellular immunity induction promoter is administered simultaneously with the HER2/neu E75 peptide and/or the modified HER2/neu E75 peptide or sequentially, and preferably, it is administered simultaneously with the peptide. The effective amount of the cellular immunity induction promoter may widely vary depending on the kind of cellular immunity induction promoter to be used, the presence or absence of other cellular immunity induction promoter and the like, and satisfactory result is obtained at a one day dose of about 0.01 µg to 1 g/kg body weight. The one day dose may be administered in a single dose or in several divided portions at several times such as two times or more, for example, two, three, four or five times. The administration interval may be arbitrary, but is appropriately selected from, for example, once per one day, once per 3 days, once per one week, once per 2 weeks, once per one month, once per 3 months, once per 6 months, once per one year, or an interval of administration longer than that, depending on the state of the patient, severity of cancer, whether it is for therapeutic purpose or preventive purpose, or the like. Generally, for the purpose of treating a patient actually having a severe cancer, the HER2/neu E75 peptide and/or modified HER2/neu E75 peptide are administered at a higher frequency and a higher dose, while for the preventive purpose for a patient having no cancer, the HER2/neu E75 peptide and/or modified HER2/neu E75 peptide are administered at a lower frequency and a lower dose.

The present invention will be explained in more detail and specifically below by way of Examples. The present invention is not limited to the Examples.

### EXAMPLES

Clofibrate: manufactured by LKT Laboratories, Inc., quercetin: manufactured by Cayman Chemical Co., resveratrol (resveratrol (synthetic)): manufactured by Wako Pure Chemical Industries, Ltd., noscapine: manufactured by Wako Pure Chemical Industries, Ltd., 3,3'-diindolylmethane: manufactured by Wako Pure Chemical Industries, Ltd., xanthone: manufactured by Wako Pure Chemical Industries, Ltd., parthenolide: manufactured by Wako Pure Chemical Industries, Ltd., etodolac: manufactured by Wako Pure Chemical Industries, Ltd., loxoprofen (loxoprofen Na): manufactured by Yoshindo, Inc., diclofenac (diclofenac sodium): manufactured by Wako Pure Chemical Industries, Ltd., ketoprofen: manufactured by Wako Pure Chemical Industries, Ltd., celecoxib: manufactured by Tocris Bioscience, docosahexaenoic acid: manufactured by Cayman Chemical Co., 2',5'-dideoxyadenosine: manufactured by Biomol International LP, SCH23390: manufactured by Wako Pure Chemical Industries, Ltd., rotigotine: manufactured by STARNASCENS, GW627368X: manufactured by Cayman Chemical Co., sulprostone: manufactured by Cayman Chemical Co. , cloprostenol: manufactured by Wako Pure Chemical Industries, Ltd., BWA868C: manufactured by Cayman Chemical Co., RO1138452: manufactured by Cayman Chemical Co., leukotriene B4: manufactured by Cayman Chemical Co., montelukast (montelukast sodium): LG Life Sciences, Ltd., zileuton: manufactured by Toronto Research Chemicals, Inc., glycyrrhizic acid (dipotassium glycyrrhizate): manufactured by Wako Pure Chemical Industries, Ltd., pirfenidone: manufactured by Tocris Bioscience, diphenhydramine (diphenhydramine hydrochloride): manufactured by Wako Pure Chemical Industries, Ltd., famotidine: manufactured by Wako Pure Chemical Industries, Ltd., immepip (immepip dihydrobromide): manufactured by Tocris Bioscience, proxyfan: manufactured by Tocris Bioscience, 4-methylhistamine: manufactured by Tocris Bioscience, olanzapine: manufactured by Wako Pure Chemical Industries, Ltd., zolmitriptan: manufactured by Cipla, Ltd., tolvaptan: manufactured by Sigma-Aldrich Co., desmopressin: manufactured by Sigma-Aldrich Co., oxybutynin (oxybutynin hydrochloride): manufactured by Sigma-Aldrich, pilocarpine (pilocarpine hydrochloride) : manufactured by Wako Pure Chemical Industries, Ltd., tamsulosin (tamsulosin hydrochloride): manufactured by Cipla, propranolol (propranolol hydrochloride): manufactured by Wako Pure Chemical Industries, Ltd., xylazine: manufactured by Wako Pure Chemical Industries, Ltd., novokinin: manufactured by Sigma-Aldrich Co., baclofen: manufactured by Tokyo Chemical Industry Co., Ltd., melatonin: manufactured by LKT Laboratories, Inc., TRAP-6: manufactured by Bachem Holding AG, adenosine diphosphate: manufactured by MP Biomedicals, LLC, somatostatin-14: manufactured by Bachem Holding AG, GW405833: manufactured by Sigma-Aldrich Co., SEW2871: manufactured by Cayman Chemical Co., trimebutine (trimebutine maleate): manufactured by Tokyo Chemical Industry Co., Ltd., loperamide (loperamide hydrochloride) : manufactured by Wako Pure Chemical Industries, Ltd., melatonin: manufactured by LKT Laboratories, Inc., L-AP4 (L-2-amino-4-phsophonobutyric acid) : manufactured by Wako Pure Chemical Industries, Ltd. , and suplatast tosylate: manufactured by TOCRIS bioscience were used.

### Liquid formulation for sublingual administration

A liquid formulation was prepared according to the formula described in the following Table 1 and used as administration samples for a mouse immunity test. Specifically, to a HER2/neu E75 peptide (TFA salt), a cellular immunity induction promoter and optionally a pharmacologically acceptable acid in the amounts described in Table 1, 20 parts by weight of an additive (DMSO) and saline as a base were added to make up 100 parts by weight in total, and the mixture was mixed. Thus, a liquid formulation for sublingual administration was prepared.

Both the HER2/neu E75 peptide and Peptide-25 were chemically synthesized and purified by HPLC. Imiquimod was purchased from Tokyo Chemical Industry Co., Ltd. Cyclic di-GMP (c-di-GMP) and cyclic di-AMP (c-di-AMP) were purchased from Biolog Life Science Institute GmbH. Pam₃CSK₄ manufactured by InvivoGen, Inc.; zymosan manufactured by Nacalai Tesque, Inc.; Poly(I:C) manufactured by InvivoGen, Inc.; Pantoea bacterium-derived lipopolysaccharide manufactured by Institute of Applied Technology for Innate Immunity; glucopyranosyl lipid manufactured by InvivoGen, Inc. (MPLAs); resiquimod (R848) and ODN1826 manufactured by InvivoGen, Inc.; pidotimod manufactured by Santa Cruz Biotechnology, Inc.; bestatine manufactured by Wako Pure Chemical Industries, Ltd.; levamisole hydrochloride manufactured by MP Biomedicals, LLC; peptidoglycan manufactured by InvivoGen, Inc.; Pam₂CSK₄ manufactured by InvivoGen, Inc.; and flagellin manufactured by InvivoGen, Inc. were used.

### Film formulation

A film formulation was prepared according to the formula described in the following Table 1. Specifically, 150 parts by weight of purified water was added to 46 parts by weight of D-mannitol (manufactured by Roquette) and 2.6 parts by weight of polyethylene glycol 400 (manufactured by Wako Pure Chemical Industries, Ltd.), and the mixture was ultrasonically stirred. To the mixture, 46 parts by weight of hydroxypropyl cellulose (manufactured by Nippon Soda Co., Ltd., HPC-SSL), 5 parts by weight of HER2/neu E75 peptide (TFA salt) (chemically synthesized and HPLC-purified product), 0.3 part by weight of Peptide-25 (chemically synthesized and HPLC-purified product), and 0.1 part by weight of a cellular immunity induction promoter other than the helper peptide were added, and the mixture was mixed under sufficient stirring. The solution in an amount equivalent to 1/100 of the original amount (2.5 parts by weight) was dropped on a peelable film made of polyethylene terephthalate, and the solution was dried in air and dried under reduced pressure to obtain 1 part by weight of film formulation. In the following mouse immunity test, each film formulation was administered in an amount of one sheet (10 mg) /time per one mouse. The sources of the cellular immunity induction promoters were the same as in the case of the liquid formulation for sublingual administration.

### Orally-disintegrating tablet

An orally-disintegrating tablet was prepared according to the formula described in the following Table 1. Specifically, 48.6 parts by weight of D-mannitol (manufactured by Roquette) and 46 parts by weight of fish-derived water-soluble gelatin were added to 400 parts by weight of purified water, and then dissolved at 35°C. To the solution, 5 parts by weight of HER2/neu E75 peptide (TFA salt) (chemically synthesized and HPLC-purified product), 0.3 part by weight of Peptide-25 (chemically synthesized and HPLC-purified product), and 0.1 part by weight of a cellular immunity induction promoter other than the helper peptide were added, and the mixture was mixed under sufficient stirring. The solution in an amount equivalent to 1/100 of the original amount (5 parts by weight) was dropped on an aluminum foil, and the solution was subjected to a freeze-drying treatment to obtain 1 part by weight of an orally-disintegrating tablet. In the mouse immunity test that will be described below, each orally-disintegrating tablet was administered in an amount of one tablet (10 mg) /time per mouse. The sources of purchase of the cellular immunity induction promoters were the same as in the case of the liquid formulation for sublingual administration.

### Mouse immunization test 1 (sublingual administration)

A mouse immunization test was carried out for the liquid formulation for sublingual administration, film formulation and orally-disintegrating tablet. The test was carried out according to an ELISPOT method. Specifically, in the case of single administration, after the mouse was anesthetized, the liquid formulation, the film formulation or the orally-disintegrating tablet was administered in the sublingual region and kept for 2 minutes, and then the mouse was reared for 6 days. In the case of double administration, the above-described operation was repeated in the same manner 6 days after the first administration. The spleen was isolated 6 days after the day of final administration, and the level of antigen-specific cellular immunity induction was evaluated by the ELISPOT method described below.

### (ELISPOT method)

A spleen cell suspension was prepared from the isolated spleen. Spleen cells (3 x 10⁶ cells/well) and the antigen peptide (100 µM) together with culturing medium were placed into a well of an ELISPOT plate on which anti-mouse IFN-γ antibody was immobilized, and the cells were co-cultured for 20 hours under the culture conditions of 37°C and 5% CO₂. The number of INF-γ producing cell spots (number of spots/3 x 10⁶ cells) was evaluated.

The results of the immunization test are shown in the following Table 1 together with the dose and the number of administrations. The mouse used was a genetically modified mouse which can be used to evaluate the cellular immunity inducing ability of a HLA-A*0201 type MHC restricted peptide. Furthermore, for a comparison, the result of the following immunization using an injectable formulation (Comparative Example 2) is described in the last part of Table 1.

**Table 1**

| | Dosage form | Composition | | | | | Dose | Administration | Results of immunization (ELISPOT average number of spots) |
|---|---|---|---|---|---|---|---|---|---|
| | | Base | Antigen peptide | Cellular immunity induction promoter | | Acid | | | |
| Comparative example 1 | liquid formulation | saline | HER2/neu E75(1.25) | None | None | None | 20 µL | 2 times | 3 |
| Example 1 | liquid formulation | saline | HER2/neu E75(1.25) | LPS(TLR4 ligand) (0.1) | None | None | 20 µL | 2 times | 15 |
| Example 2 | liquid formulation | saline | HER2/neu E75(1.25) | None | PEP(0.3) | None | 20 µL | 2 times | 11 |
| Example 3 | liquid formulation | saline | HER2/neu E75(1.25) | LPS (TLR4 ligand) (0.1) | PEP(0.3) | None | 20 µL | 2 times | 30 |
| Example 4 | liquid formulation | saline | HER2/neu E75(1.25) | LPS(TLR4 ligand) (0.1) | PEP(0.3) | MA(0.05) | 20 µL | 2 times | 41 |
| Example 5 | liquid formulation | saline | HER2/neu E75(1.25) | LPS(TLR4 ligand) (0.1) | PEP(0.3) | isostearic acid (0.05) | 20 µL | 2 times | 38 |
| Example 6 | liquid formulation | saline | HER2/neu E75(1.25) | LPS(TLR4 ligand) (0.1) | PEP(0.3) | lactic acid (0.05) | 20 µL | 2 times | 37 |
| Example 7 | liquid formulation | saline | HER2/neu E75(1.25) | LPS(TLR4 ligand) (0.1) | PEP(0.3) | citric acid (0.05) | 20 µL | 2 times | 38 |
| Example 8 | liquid formulation | saline | HER2/neu E75(1.25) | syn-MPL(TLR4 ligand) (0.1) | PEP(0.3) | None | 20 µL | 2 times | 27 |
| Example 9 | liquid formulation | saline | HER2/neu E75(1.25) | imiquimod (TLR7 and/or TLR8 ligand) (0.3) | PEP(0.3) | None | 20 µL | 2 times | 89 |
| Example 10 | liquid formulation | saline | HER2/neu E75(1.25) | resiquimod (TLR7 and/or TLR8 ligand) (0.1) | PEP(0.3) | None | 20 µL | 2 times | 177 |
| Example 11 | liquid formulation | saline | HER2/neu E75(1.25) | c-di-GMP (cyclic dinucleotide) (0.2) | None | None | 10 µL | 1 time | 523 |
| Example 12 | liquid formulation | saline | HER2/neu E75(1.25) | c-di-GMP (cyclic dinucleotide) (0.2) | PEP(0.3) | None | 10 µL | 1 time | 611 |
| Example 13 | liquid formulation | saline | HER2/neu E75(1.25) | levamisole HCl (immunomodulato ry small molecule drug) (0.5) | None | None | 20 µL | 2 times | 45 |
| Example 14 | liquid formulation | saline | HER2/neu E75(1.25) | levamisole HCl (immunomodul atory small molecule drug) (0.5) | PEP(0.3) | None | 20 µL | 2 times | 70 |
| Example 15 | liquid formulation | saline | HER2/neu E75(1.25) | Bestatin(immuno modulatory small molecule drug) (0.5) | PEP(0.3) | None | 20 µL | 2 times | |
| Example 16 | liquid formulation | saline | HER2/neu E75(1.25) | pidotimod(immun omodulatory small molecule drug) (0.5) | PEP(0.3) | None | 20 µL | 2 times | |
| Example 17 | liquid formulation | saline | HER2/neu E75(1.25) | clofibrate(PPAR agonist) (0.1) | PEP(0.3) | None | 20 µL | 2 times | 85 |
| Example 18 | liquid formulation | saline | HER2/neu E75(1.25) | quercetin(TSLP production inhibitor) (0.1) | None | None | 20 µL | 2 times | 67 |
| Example 19 | liquid formulation | saline | HER2/neu E75(1.25) | quercetin(TSLP production inhibitor) (0.1) | PEP(0.3) | None | 20 µL | 2 times | 94 |
| Example 20 | liquid formulation | saline | HER2/neu E75(1.25) | noscapine(TSLP production inhibitor) (0.1) | PEP(0.3) | None | 20 µL | 2 times | 78 |
| Example 21 | liquid formulation | saline | HER2/neu E75(1.25) | 3,3'-diindolylm ethane (TSLP production inhibitor) (0.1) | PEP(0.3) | None | 20 µL | 2 times | |
| Example 22 | liquid formulation | saline | HER2/neu E75(1.25) | xanthone(TSLP production inhibitor) (0.1) | PEP(0.3) | None | 20 µL | 2 times | 110 |
| Example 23 | liquid formulation | saline | HER2/neu E75(1.25) | parthenolide(TS LP production inhibitor) (0.1) | PEP(0.3) | None | 20 µL | 2 times | 105 |
| Example 24 | liquid formulation | saline | HER2/neu E75(1.25) | loxoprofen(COX inhibitor) (0.1) | None | None | 20 µL | 2 times | 56 |
| Example 25 | liquid formulation | saline | HER2/neu E75 (1.25) | loxoprofen(COX inhibitor) (0.1) | PEP(0.3) | None | 20 µL | 2 times | 89 |
| Example 26 | liquid formulation | saline | HER2/neu E75(1.25) | etodolac(COX inhibitor) (0.1) | PEP(0.3) | None | 20 µL | 2 times | 78 |
| Example 27 | liquid formulation | saline | HER2/neu E75(1.25) | diclofenac(COX inhibitor) (0.1) | PEP(0.3) | None | 20 µL | 2 times | |
| Example 28 | liquid formulation | saline | HER2/neu E75(1.25) | ketoprofen(COX inhibitor) (0.1) | PEP(0.3) | None | 20 µL | 2 times | |
| Example 29 | liquid formulation | saline | HER2/neu E75(1.25) | celecoxib(COX inhibitor) (0.1) | PEP(0.3) | None | 20 µL | 2 times | 123 |
| Example 30 | liquid formulation | saline | HER2/neu E75(1.25) | docosahexaenoic acid(omega-3 fatty acid) (0.1) | PEP(0.3) | None | 20 µL | 2 times | |
| Example 31 | liquid formulation | saline | HER2/neu E75(1.25) | 2',5'-dideoxyad enosine(adenyla te cyclase inhibitor) (0.1) | PEP(0.3) | None | 20 µL | 2 times | |
| Example 32 | liquid formulation | saline | HER2/neu E75(1.25) | SCH23390 (dopamine receptor antagonist) (0.1) | PEP(0.3) | None | 20 µL | 2 times | |
| Example 33 | liquid formulation | saline | HER2/neu E75(1.25) | rotigotine(dopa mine receptor agonist) (0.1) | PEP(0.3) | None | 20 µL | 2 times | |
| Example 34 | liquid formulation | saline | HER2/neu E75(1.25) | GW627368X(prost aglandin receptor antagonist) (0.1) | PEP(0.3) | None | 20 µL | 2 times | 85 |
| Example 35 | liquid formulation | saline | HER2/neu E75(1.25) | sulprostone(pro staglandin receptor agonist) (0.1) | PEP(0.3) | None | 20 µL | 2 times | 89 |
| Example 36 | liquid formulation | saline | HER2/neu E75(1.25) | BWA868C (prostaglandin receptor antagonist) (0.1) | PEP(0.3) | None | 20 µL | 2 times | 76 |
| Example 37 | liquid formulation | saline | HER2/neu E75(1.25) | RO1138452 (prostaglandin receptor antagonist) (0.1) | PEP(0.3) | None | 20 µL | 2 times | 87 |
| Example 38 | liquid formulation | saline | HER2/neu E75(1.25) | montelukast(leu kotriene receptor antagonist) (0.1) | PEP(0.3) | None | 20 µL | 2 times | |
| Example 39 | liquid formulation | saline | HER2/neu E75(1.25) | zileuton(leukot riene receptor antagonist) (0.1) | PEP(0.3) | None | 20 µL | 2 times | |
| Example 40 | liquid formulation | saline | HER2/neu E75(1.25) | dipotassium glycyrrhizinate (phospholipase A2 inhibitor) (0.1) | PEP(0.3) | None | 20 µL | 2 times | |
| Example 41 | liquid formulation | saline | HER2/neu E75(1.25) | pirfenidone(TGF -beta production inhibitor) (0.1) | PEP(0.3) | None | 20 µL | 2 times | |
| Example 42 | liquid formulation | saline | HER2/neu E75(1.25) | diphenhydramine (histamine receptor antagonist) (0.1) | PEP(0.3) | None | 20 µL | 2 times | 76 |
| Example 43 | liquid formulation | saline | HER2/neu E75 (1.25) | famotidine(hist amine receptor antagonist) (0.1) | PEP(0.3) | None | 20 µL | 2 times | 86 |
| Example 44 | liquid formulation | saline | HER2/neu E75(1.25) | proxyfan(histam ine receptor agonist) (0.1) | PEP(0.3) | None | 20 µL | 2 times | 65 |
| Example 45 | liquid formulation | saline | HER2/neu E75(1.25) | 4-methylhistami ne(histamine receptor agonist) (0.1) | PEP(0.3) | None | 20 µL | 2 times | |
| Example 46 | liquid formulation | saline | HER2/neu E75(1.25) | olanzapine(sero tonin receptor antagonist) (0.1) | PEP(0.3) | None | 20 µL | 2 times | 55 |
| Example 47 | liquid formulation | saline | HER2/neu E75(1.25) | zolmitriptan(se rotonin receptor agonist) (0.1) | PEP(0.3) | None | 20 µL | 2 times | 95 |
| Example 48 | liquid formulation | saline | HER2/neu E75(1.25) | tolvaptan(vasop ressin receptor antagonist) (0.1) | PEP(0.3) | None | 20 µL | 2 times | |
| Example 49 | liquid formulation | saline | HER2/neu E75(1.25) | desmopressin(va sopressin receptor agonist) (0.1) | PEP(0.3) | None | 20 µL | 2 times | |
| Example 50 | liquid formulation | saline | HER2/neu E75(1.25) | oxybutynin(musc arine receptor antagonist) (0.1 ) | PEP(0.3) | None | 20 µL | 2 times | 56 |
| Example 51 | liquid formulation | saline | HER2/neu E75(1.25) | pilocarpine(mus carine receptor agonist) (0.1) | PEP(0.3) | None | 20 µL | 2 times | |
| Example 52 | liquid formulation | saline | HER2/neu E75(1.25) | tamsulosin(adre nalin receptor antagonist) (0.1) | PEP(0.3) | None | 20 µL | 2 times | 67 |
| Example 53 | liquid formulation | saline | HER2/neu E75(1.25) | propranolol(adr enalin receptor antagonist) (0.1) | PEP(0.3) | None | 20 µL | 2 times | 67 |
| Example 54 | liquid formulation | saline | HER2/neu E75(1.25) | xylazine(adrena lin receptor agonist) (0.1) | PEP(0.3) | None | 20 µL | 2 times | |
| Example 55 | liquid formulation | saline | HER2/neu E75(1.25) | novokinin(angio tensin receptor agonist) (0.1) | PEP(0.3) | None | 20 µL | 2 times | |
| Example 56 | liquid formulation | saline | HER2/neu E75(1.25) | baclofen(GABA receptor agonist) (0.1) | PEP(0.3) | None | 20 µL | 2 times | |
| Example 57 | liquid formulation | saline | HER2/neu E75(1.25) | melatonin(melat onin receptor agonist) (0.1) | PEP(0.3) | None | 20 µL | 2 times | 76 |
| Example 58 | liquid formulation | saline | HER2/neu E75(1.25) | adenosine diphosphate(ADP receptor agonist) (0.1) | PEP(0.3) | None | 20 µL | 2 times | |
| Example 59 | liquid formulation | saline | HER2/neu E75(1.25) | trimebutine(mus carine receptor antagonist) (0.1) | PEP(0.3) | None | 20 µL | 2 times | |
| Example 60 | liquid formulation | saline | HER2/neu E75(1.25) | L-AP4(metabotro pic glutamate receptor agonist) (0.1) | PEP(0.3) | None | 20 µL | 2 times | 67 |
| Example 61 | liquid formulation | saline | HER2/neu E75(1.25) | TRAP-6(thrombin receptor agonist) (0.1) | PEP(0.3) | None | 20 µL | 2 times | |
| Example 62 | liquid formulation | saline | HER2/neu E75(1.25) | loperamide (opioid receptor agonist) (0.1) | PEP(0.3) | None | 20 µL | 2 times | 76 |
| Example 63 | liquid formulation | saline | HER2/neu E75(1.25) | leukotriene B4(leukotriene receptor agonist) (0.1) | PEP(0.3) | None | 20 µL | 2 times | |
| Example 64 | liquid formulation | saline | HER2/neu E75(1.25) | somatostatin-14 (somatostatin receptor agonist) (0.1) | PEP(0.3) | None | 20 µL | 2 times | |
| Example 65 | liquid formulation | saline | HER2/neu E75(1.25) | GW405833 (cannabinoid receptor agonist) (0.1) | PEP(0.3) | None | 20 µL | 2 times | |
| Example 66 | liquid formulation | saline | HER2/neu E75(1.25) | SEW2871(sphingo sine-1 phosphate receptor agonist) (0.1) | PEP(0.3) | None | 20 µL | 2 times | |
| Example 67 | liquid formulation | saline | HER2/neu E75(1.25) | suplatast tosylate(Th2 cytokine inhibitor) (0.1) | PEP(0.3) | None | 20 µL | 2 times | |
| Example 68 | liquid formulation | saline | HER2/neu E75(1.25) | LPS(TLR4 ligand) (0.1) | PADRE (0.3) | None | 20 µL | 2 times | 33 |
| Example 69 | film formulation | HPC/PEG/ mannitol | HER2/neu E75(5) | LPS(TLR4 ligand) (0.1) | PEP(0.3) | None | 10 mg | 2 times | 32 |
| Example 70 | film formulation | HPC/PEG/ mannitol | HER2/neu E75 (5) | c-di-GMP(cyclic dinucleotide) (0.1) | PEP(0.3) | None | 10 mg | 2 times | 651 |
| Example 71 | film formulation | HPC/PEG/ mannitol | HER2/neu E75(5) | quercetin(TSLP production inhibitor) (0.1) | PEP(0.3) | None | 10 mg | 2 times | 104 |
| Example 72 | film formulation | HPC/PEG/ mannitol | HER2/neu E75(5) | loxoprofen(COX inhibitor) (0.1) | PEP(0.3) | None | 10 mg | 2 times | 95 |
| Example 73 | orally-disin tegrating tablet | gelatin/ mannitol | HER2/neu E75(5) | LPS(TLR4 ligand) (0.1) | PEP(0.3) | None | 10 mg | 2 times | 33 |
| Example 74 | orally-disin tegrating tablet | gelatin/ mannitol | HER2/neu E75(5) | c-di-GMP(cyclic dinucleotide) (0.1) | PEP(0.3) | None | 10 mg | 2 times | 636 |
| Example 75 | orally-disin tegrating tablet | gelatin/ mannitol | HER2/neu E75(5) | quercetin(TSLP production inhibitor) (0.1) | PEP(0.3) | None | 10 mg | 2 times | 101 |
| Example 76 | orally-disin tegrating tablet | gelatin/ mannitol | HER2/neu E75(5) | loxoprofen(COX. inhibitor) (0.1) | PEP(0.3) | None | 10 mg | 2 times | 97 |
| Comparative example 2 | subcutaneous injection | saline | HER2/neu E75 (0.125) | Montanide ISA51VG (50) | | None | 200 µL | 1 time | 110 |

| | | | | | | | | | |
|---|---|---|---|---|---|---|---|---|---|
| Values in the parentheses indicate the mixing proportions (parts by weight) of the respective components (The same also applies to the following tables). HPC: Hydroxypropyl cellulose PEG: Polyethylene glycol 400 LPS: Pantoea bacterium-derived polysaccharide syn-MPL: Synthetic monophospholipid A (glucopyranosyl lipid) c-di-GMP: Cyclic di-GMP Levamisole HCl: Levamisole hydrochloride PEP: Peptide-25 (SEQ ID NO: 3) (helper peptide) PADRE: PADRE (SEQ ID NO: 4) (helper peptide) MA: Myristic acid | | | | | | | | | |

### Liquid formulation for nasal administration

A liquid formulation was prepared according to the formula described in the following Table 2 and used as administration sample for a mouse immunization test. Specifically, to a HER2/neu E75 peptide and a cellular immunity induction promoter in the amounts described in Table 2, 20 parts by weight of additive (DMSO) and saline as a base were added to make up 100 parts by weight in total, and then mixed. Thus, a liquid formulation for nasal administration was prepared. The sources of the HER2/neu E75 peptide and the cellular immunity induction promoters were the same as in the case of the liquid formulation for sublingual administration.

### Mouse immunization test 2 (nasal administration)

A mouse immunization test was carried out for the liquid formulation for nasal administration described above. The test was carried out according to an ELISPOT method. Specifically, in the case of single administration, after the mouse was anesthetized, the liquid formulation was administered by inhalation through the nasal cavity, and the mouse was reared for 6 days. In the case of double administration, the operation described above was repeated in the same manner 6 days after the first administration. The spleen was isolated 6 days after the day of final administration, and the level of antigen-specific cellular immunity induction was evaluated by an ELISPOT method. The ELISPOT method was carried out by the same method as that used in the mouse immunity test 1.

The results of the immunization test are shown in the following Table 2 together with the dose and the number of administrations. The mouse used was a genetically modified mouse which can be used to evaluate the cellular immunity inducing ability of a HLA-A*0201 type MHC restricted peptide. Furthermore, for a comparison, the result of the following immunization using an injectable formulation (Comparative Example 2) is described in the last part of Table 2.

**Table 2**

| | Dosage form | Composition | | | | Dose | Administration | Results of immunization (ELISPOT average number of spots) |
|---|---|---|---|---|---|---|---|---|
| | | Base | Antigen peptide | Cellular immunity induction promoter | | | | |
| Comparative example 3 | liquid formulation | saline | HER2/neu E75(1.25) | None | None | 10 µL | 2 times | 3 |
| Example 77 | liquid formulation | saline | HER2/neu E75(1.25) | None | PEP(0.3) | 10 µL | 2 times | 11 |
| Example 78 | liquid formulation | saline | HER2/neu E75(1.25) | LPS (TLR4 ligand) (0.1) | None | 10 µL | 2 times | 16 |
| Example 79 | liquid formulation | saline | HER2/neu E75(1.25) | LPS (TLR4 ligand) (0.1) | PEP(0.3) | 10 µL | 2 times | 31 |
| Example 80 | liquid formulation | saline | HER2/neu E75(1.25) | Pam3CSK4 (TLR1/2 ligand) (0.1) | PEP(0.3) | 10 µL | 2 times | 69 |
| Example 81 | liquid formulation | saline | HER2/neu E75(1.25) | poly(I:C)(TLR3 ligand) (0.1) | PEP(0.3) | 10 µL | 2 times | 183 |
| Example 82 | liquid formulation | saline | HER2/neu E75(1.25) | c-di-GMP(cyclic dinucleotide) (0.2) | PEP(0.3) | 10 µL | 1 time | 476 |
| Example 83 | liquid formulation | saline | HER2/neu E75(1.25) | clofibrate(PPAR agonist) (0.1) | PEP(0.3) | 10 µL | 2 times | 87 |
| Example 84 | liquid formulation | saline | HER2/neu E75(1.25) | quercetin(TSLP production inhibitor) (0.1) | None | 10 µL | 2 times | |
| Example 85 | liquid formulation | saline | HER2/neu E75(1.25) | quercetin(TSLP production inhibitor) (0.1) | PEP(0.3) | 10 µL | 2 times | |
| Example 86 | liquid formulation | saline | HER2/neu E75(1.25) | resveratrol (TSLP production inhibitor) (0.1) | PEP(0.3) | 10 µL | 2 times | 132 |
| Example 87 | liquid formulation | saline | HER2/neu E75(1.25) | loxoprofen(COX inhibitor) (0.1) | None | 10 µL | 2 times | 89 |
| Example 88 | liquid formulation | saline | HER2/neu E75 (1.25) | loxoprofen(COX inhibitor) (0.1) | PEP(0.3) | 10 µL | 2 times | 125 |
| Example 89 | liquid formulation | saline | HER2/neu E75(1.25) | 2',5'-dideoxyad enosine(adenyla te cyclase inhibitor) (0.1) | PEP(0.3) | 10 µL | 2 times | |
| Example 90 | liquid formulation | saline | HER2/neu E75(1.25) | SCH23390 (dopamine receptor antagonist) (0.1) | PEP(0.3) | 10 µL | 2 times | |
| Example 91 | liquid formulation | saline | HER2/neu E75 (1.25) | rotigotine (dopamine receptor agonist) (0.1) | PEP(0.3) | 10 µL | 2 times | |
| Example 92 | liquid formulation | saline | HER2/neu E75(1.25) | GW627368X (prostaglandin receptor antagonist) (0.1) | PEP(0.3) | 10 µL | 2 times | 93 |
| Example 93 | liquid formulation | saline | HER2/neu E75(1.25) | sulprostone(pro staglandin receptor agonist) (0.1) | PEP(0.3) | 10 µL | 2 times | 114 |
| Example 94 | liquid formulation | saline | HER2/neu E75(1.25) | cloprostenol(pr ostaglandin receptor agonist) (0.1) | PEP(0.3) | 10 µL | 2 times | |
| Example 95 | liquid formulation | saline | HER2/neu E75 (1.25) | montelukast(leu kotriene receptor antagonist) (0.1) | PEP(0.3) | 10 µL | 2 times | |
| Example 96 | liquid formulation | saline | HER2/neu E75(1.25) | dipotassium glycyrrhizinate (phospholipase A2 inhibitor)(0.1) | PEP(0.3) | 10 µL | 2 times | |
| Example 97 | liquid formulation | saline | HER2/neu E75(1.25) | pirfenidone(TGF -beta production inhibitor) (0.1) | PEP(0.3) | 10 µL | 2 times | |
| Example 98 | liquid formulation | saline | HER2/neu E75(1.25) | diphenhydramine (histamine receptor antagonist) (0.1) | PEP(0.3) | 10 µL | 2 times | |
| Example 99 | liquid formulation | saline | HER2/neu E75(1.25) | famotidine(hist amine receptor antagonist) (0.1) | PEP(0.3) | 10 µL | 2 times | 84 |
| Example 100 | liquid formulation | saline | HER2/neu E75(1.25) | proxyfan(histam ine receptor agonist) (0.1) | PEP(0.3) | 10 µL | 2 times | |
| Example 101 | liquid formulation | saline | HER2/neu E75 (1.25) | olanzapine(sero tonin receptor antagonist) (0.1) | PEP(0.3) | 10 µL | 2 times | |
| Example 102 | liquid formulation | saline | HER2/neu E75(1.25) | zolmitriptan(se rotonin receptor agonist) (0.1) | PEP(0.3) | 10 µL | 2 times | 65 |
| Example 103 | liquid formulation | saline | HER2/neu E75(1.25) | tolvaptan(vasop ressin receptor antagonist) (0.1) | PEP(0.3) | 10 µL | 2 times | |
| Example 104 | liquid formulation | saline | HER2/neu E75(1.25) | desmopressin(va sopressin receptor agonist) (0.1) | PEP(0.3) | 10 µL | 2 times | |
| Example 105 | liquid formulation | saline | HER2/neu E75(1.25) | pilocarpine(mus carine receptor agonist) (0.1) | PEP(0.3) | 10 µL | 2 times | |
| Example 106 | liquid formulation | saline | HER2/neu E75(1.25) | tamsulosin(adre nalin receptor antagonist) (0.1) | PEP(0.3) | 10 µL | 2 times | |
| Example 107 | liquid formulation | saline | HER2/neu E75(1.25) | propranolol(adr enalin receptor antagonist) (0.1) | PEP(0.3) | 10 µL | 2 times | |
| Example 108 | liquid formulation | saline | HER2/neu E75(1.25) | xylazine(adrena lin receptor agonist) (0.1) | PEP(0.3) | 10 µL | 2 times | |
| Example 109 | liquid formulation | saline | HER2/neu E75(1.25) | melatonin(melat onin receptor agonist) (0.1) | PEP(0.3) | 10 µL | 2 times | 76 |
| Example 110 | liquid formulation | saline | HER2/neu E75(1.25) | novokinin(angio tensin receptor agonist) (0.1) | PEP(0.3) | 10 µL | 2 times | |
| Example 111 | liquid formulation | saline | HER2/neu E75(1.25) | baclofen(GABA receptor agonist) (0.1) | PEP(0.3) | 10 µL | 2 times | |
| Example 112 | liquid formulation | saline | HER2/neu E75 (1.25) | trimebutine(mus carine receptor antagonist) (0.1) | PEP(0.3) | 10 µL | 2 times | |
| Example 113 | liquid formulation | saline | HER2/neu E75(1.25) | L-AP4(metabotro pic glutamate receptor agonist) (0.1) | PEP(0.3) | 10 µL | 2 times | 56 |
| Example 114 | liquid formulation | saline | HER2/neu E75(1.25) | TRAP-6 (thrombin receptor agonist) (0.1) | PEP(0.3) | 10 µL | 2 times | |
| Example 115 | liquid formulation | saline | HER2/neu E75(1.25) | loperamide(opio id receptor agonist) (0.1) | PEP(0.3) | 10 µL | 2 times | 67 |
| Example 116 | liquid formulation | saline | HER2/neu E75(1.25) | leukotriene B4(leukotriene receptor agonist) (0.1) | PEP(0.3) | 10 µL | 2 times | |
| Example 117 | liquid formulation | saline | HER2/neu E75(1.25) | somatostatin-14 (somatostatin receptor agonist) (0.1) | PEP(0.3) | 10 µL | 2 times | |
| Example 118 | liquid formulation | saline | HER2/neu E75(1.25) | GW405833 (cannabinoid receptor agonist) (0.1) | PEP(0.3) | 10 µL | 2 times | |
| Example 119 | liquid formulation | saline | HER2/neu E75(1.25) | SEW2871(sphingo sine-1 phosphate receptor agonist) (0.1) | PEP(0.3) | 10 µL | 2 times | |
| Example 120 | liquid formulation | saline | HER2/neu E75(1.25) | suplatast tosylate(Th2 cytokine inhibitor) (0.1) | PEP(0.3) | 10 µL | 2 times | |
| Example 121 | liquid formulation | saline | HER2/neu E75(1.25) | adenosine diphosphate(ADP receptor agonist) (0.1) | PEP(0.3) | 10 µL | 2 times | |
| Example 122 | liquid formulation | saline | HER2/neu E75(1.25) | LPS (TLR4 ligand) (0.1) | PADRE (0.3) | 10 µL | 2 times | 34 |
| Comparative example 2 | subcutaneous injection | saline | HER2/neu E75 (0.125) | Montanide ISA51VG (50) | | 200 µL | 1 time | 110 |

| | | | | | | | | |
|---|---|---|---|---|---|---|---|---|
| Poly(I:C): Polyinosinic-polycytidylic acid | | | | | | | | |

### Subcutaneous injectable formulation

A subcutaneous injectable formulation was prepared according to the formula described in the following Table 3 and used as the administration sample for an immunization test. Specifically, to the HER2/neu E75 peptide and Montanide ISA51VG (Freund Corporation) as an adjuvant in the amounts described in Table 3, 0.5 part by weight of additive (DMSO) and saline as a base were added to make up 100 parts by weight in total, and then mixed to prepare an injectable formulation. The source of the HER2/neu E75 peptide was the same as in the case of the liquid formulation for sublingual administration.

### Mouse immunization test 3 (subcutaneous injection)

A mouse immunization test was carried out for the subcutaneous injectable formulation described above. The test was carried out according to an ELISPOT method. Specifically, 200 µL of the injectable formulation was administered by subcutaneous injection to the dorsal side of a mouse, and then the mouse was reared for 6 days. The spleen was isolated 6 days after the day of administration, and the level of antigen-specific cellular immunity induction was evaluated by an ELISPOT method. The number of administration was set to once. The mouse used was a genetically modified mouse which can be used to evaluate the cellular immunity inducing ability of a HLA-A*0201 type MHC-restricted peptide can be evaluated. The ELISPOT method was carried out by the same method as that used in the mouse immunization test 1. The results of the immunization test are shown in the following Table 3.

**Table 3**

| | Composition | | | Result of immunization (ELISPOT average number of spots) |
|---|---|---|---|---|
| | Base | Antigen peptide | Cellular immunity induction promoter | |
| Comparative Example 2 | saline | HER2/neu E75 (0.125) | Montanide ISA51VG(50) | 110 |

A cancer vaccine composition for mucosal administration comprising HER2/neu E75 peptide and/or a modified HER2/neu E75 peptide and a first cellular immunity induction promoter was administered in sublingual (Table 1) or nasal (Table 2) route, and an efficacy of the first cellular immunity induction promoter was evaluated.

As a result, a cellular immunity induction promoter selected from TLR ligand, cyclic dinucleotide, helper peptide, immunomodulatory small molecule drug, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor, Th2 cytokine inhibitor and a combination of two or more kinds of them, was preferable.

Preferably, a first cellular immunity induction promoter selected from TLR ligand, cyclic dinucleotide, immunomodulatory small molecule drug, cyclooxygenase inhibitor, TSLP production inhibitor, prostaglandin receptor antagonist, prostaglandin receptor agonist, PPAR agonist, TGF-beta production inhibitor, histamine receptor agonist, histamine receptor antagonist, serotonin receptor agonist, serotonin receptor antagonist, muscarine receptor antagonist, adrenalin receptor antagonist, opioid receptor agonist, melatonin receptor agonist, metabotropic glutamate receptor agonist and a combination of two or more kinds of them, as well as a combination of a helper peptide with the first cellular immunity induction promoter other than helper peptide were effective.

Regarding sublingual administration, more preferably, a first cellular immunity induction promoter selected from TLR4 ligand, TLR7 and/or TLR8 ligand, cyclic dinucleotide, immunomodulatory small molecule drug, cyclooxygenase inhibitor, TSLP production inhibitor, prostaglandin receptor antagonist, prostaglandin receptor agonist, PPAR agonist, TGF-beta production inhibitor, histamine receptor agonist, histamine receptor antagonist, serotonin receptor agonist, serotonin receptor antagonist, muscarine receptor antagonist, adrenalin receptor antagonist, opioid receptor agonist, melatonin receptor agonist, metabotropic glutamate receptor agonist and a combination of two or more kinds of them, as well as a combination of a helper peptide with the first cellular immunity induction promoter other than helper peptide were effective.

Regarding nasal administration, further preferably, a first cellular immunity induction promoter selected from TLR4 ligand, TLR1/2 ligand, TLR3 ligand, cyclic dinucleotide, immunomodulatory small molecule drug, cyclooxygenase inhibitor, TSLP production inhibitor, prostaglandin receptor antagonist, prostaglandin receptor agonist, PPAR agonist, TGF-beta production inhibitor, histamine receptor agonist, histamine receptor antagonist, serotonin receptor agonist, serotonin receptor antagonist, muscarine receptor antagonist, adrenalin receptor antagonist, opioid receptor agonist, melatonin receptor agonist, metabotropic glutamate receptor agonist and a combination of two or more kinds of them, as well as a combination of a helper peptide with the first cellular immunity induction promoter other than helper peptide were effective.

It was also confirmed that the induction of cellular immunity is promoted by the addition of a pharmacologically acceptable acid or a pharmacologically acceptable salt thereof as a second cellular immunity induction promoter.

From the viewpoint of safety, a sublingual administration is preferable rather than a nasal administration. However, the strong induction of immunity was also confirmed when a film formulation or an orally-disintegrating tablet, which is a preferable form in view of convenient administration and storage stability, was used.

## Claims

1. A cancer vaccine composition for mucosal administration for use in the treatment of a cancer, comprising:
(i) a HER2/neu E75 peptide and/or a modified HER2/neu E75 peptide; and
(ii) a first cellular immunity induction promoter selected from a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenaline receptor antagonist, an adrenaline receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor, a Th2 cytokine inhibitor, and combinations of two or more kinds thereof.

2. The cancer vaccine composition for mucosal administration according to claim 1, further comprising a second cellular immunity induction promoter that is a pharmacologically acceptable acid or a pharmacologically acceptable salt thereof.

3. The cancer vaccine composition for mucosal administration according to claim 1 or 2, wherein the first cellular immunity induction promoter is a helper peptide.

4. The cancer vaccine composition for mucosal administration according to claim 1 or 2, wherein the first cellular immunity induction promoter is a combination of a helper peptide and at least one substance selected from the group consisting of a TLR ligand, a cyclic dinucleotide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenaline receptor antagonist, an adrenaline receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor and a Th2 cytokine inhibitor.

5. The cancer vaccine composition according to any one of claims 1 to 4, which is provided in the form of a film preparation.

6. The cancer vaccine composition according to any one of claims 1 to 4, which is provided in the form of a liquid formulation.

7. The cancer vaccine composition according to any one of claims 1 to 4, which is provided in the form of an orally-disintegrating tablet for mucosal administration.
